(19) 

Europäisches Patentamt
European Patent Office
Office européen des brevets

(11) **EP 4 495 839 A1**

(12) **EUROPEAN PATENT APPLICATION**
published in accordance with Art. 153(4) EPC

(43) Date of publication:
**22.01.2025 Bulletin 2025/04**

(21) Application number: **22931988.4**

(22) Date of filing: **15.03.2022**

(51) International Patent Classification (IPC):
***G06N 10/20*** (2022.01)

(52) Cooperative Patent Classification (CPC):
**G06N 10/20**

(86) International application number:
**PCT/JP2022/011510**

(87) International publication number:
**WO 2023/175703 (21.09.2023 Gazette 2023/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **FUJITSU LIMITED**
**Kawasaki-shi, Kanagawa 211-8588 (JP)**

(72) Inventor: **MORITA, Mikio**
**Kawasaki-shi, Kanagawa 211-8588 (JP)**

(74) Representative: **Haseltine Lake Kempner LLP**
**Cheapside House**
**138 Cheapside**
**London EC2V 6BJ (GB)**

(54) **INFORMATION PROCESSING PROGRAM, INFORMATION PROCESSING METHOD, AND INFORMATION PROCESSING DEVICE**

(57)     The number of gate operations is to be reduced.
An information processing device (10) calculates an initial value of a variable corresponding to each electron excitation of a plurality of electron excitations to be generated in quantum bits to calculate the base energy of a molecule by the variational quantum eigensolver. Next, for each electron excitation of the plurality of electron excitations, the information processing device (10) determines whether to implement the corresponding circuit, based on whether the initial value of the corresponding variable is equal to or greater than a predetermined threshold. The information processing device (10) then disposes, in a quantum circuit (1) for calculating the base energy of a molecule, partial circuits that perform electron excitation operations for calculating the energy in accordance with the value of the variable, for each of the electron excitations for which it has been determined to implement the corresponding circuits among the plurality of electron excitations.

FIG. 1

**Description**

TECHNICAL FIELD

The present invention relates to an information processing program, an information processing method, and an information processing device.

BACKGROUND ART

In the field of quantum computers, noisy intermediate-scale quantum computers (NISQ) are expected to be put into practical use. A NISQ is a medium-scale quantum computer without an error correction function. One application of a NISQ is calculation by the variational quantum eigensolver (VQE). The VQE is a variational algorithm for obtaining a ground state of a quantum many-body system. The VQE may be used for performing quantum chemical calculation in a NISQ, for example. The quantum chemical calculation is calculation for obtaining a molecular state and physical property information by solving a Schrödinger equation. At present, various studies are in progress for putting calculation using the VQE into practical use.

As a technique related to quantum calculation such as the VQE, there is a proposed quantum information processing method for obtaining a differential of energy, by which a derivative of energy can be obtained when quantum calculation of the energy of a system is performed with the VQE, for example. Furthermore, there also is a proposed method that uses classical data in quantum operations by inputting the classical data as quantum data. Further, a system for implementing a high-hardware-efficiency variational quantum eigensolver for quantum computing machines has also been proposed. Also, VQE Ansatz with less complicated quantum gates has been proposed.

CITATION LIST

PATENT DOCUMENTS

Patent Document 1: International Publication Pamphlet No. WO 2020/230794
Patent Document 2: U.S. Patent Application Publication No. 2019/0354316
Patent Document 3: Japanese National Publication of International Patent Application No. 2020-534607

NON-PATENT DOCUMENT

Non-Patent Document 1: Rongxin Xia, Sabre Kais, "Qubit coupled cluster singles and doubles variational quantum eigensolver ansatz for electronic structure calculations" arXiv:2005.08451v3, 10 Oct 2020

SUMMARY OF INVENTION

TECHNICAL PROBLEM

One of the problems for practical use of quantum chemical calculation by the VQE lies in that the number of gate operations is large. If the number of gate operations is too large, the calculation time increases, calculation is not completed within the quantum bit duration (coherence time), and any calculation result might not be obtained. Furthermore, when the number of gate operations is large, errors accumulate during the quantum calculation process, and the influence of the errors on calculation results becomes larger.

In one aspect, an object of the present invention is to reduce the number of gate operations.

SOLUTION TO PROBLEM

In one proposal, an information processing program for causing a computer to perform the following process is provided.

The computer calculates an initial value of a variable corresponding to each electron excitation of a plurality of electron excitations for calculating the base energy of a molecule by the variational quantum eigensolver. For each electron excitation of the plurality of electron excitations, the computer determines whether to implement the corresponding circuit, based on whether the initial value of the corresponding variable is greater than a predetermined threshold. The computer then disposes, in a quantum circuit for calculating the base energy of a molecule, partial circuits that perform electron excitation gate operations for calculating the energy in accordance with the value of the variable, for each of the electron excitations for which it has been determined to implement the corresponding circuits among the plurality of electron excitations.

ADVANTAGEOUS EFFECTS OF INVENTION

According to one aspect, the number of gate operations can be reduced.

The object described above and other objects, features, and advantages of the present invention will become clear from the following description related to the appended drawings, which represent preferred embodiments as examples of the present invention.

BRIEF DESCRIPTION OF DRAWINGS

FIG. 1 is a diagram illustrating an example of an information processing method according to a first embodiment.
FIG. 2 is a diagram illustrating an example of a system configuration according to a second embodiment.
FIG. 3 is a diagram illustrating an example of the hardware of a classical computer.
FIG. 4 is a block diagram illustrating an example of functions of a classical computer for quantum chemical calculation by the VQE.
FIG. 5 is a diagram for explaining an Ansatz circuit.
FIG. 6 is a diagram illustrating an example of a reduction in the number of gates with rotation gate elements.
FIG. 7 is a diagram illustrating an example of a Givens rotation circuit.
FIG. 8 is a diagram illustrating an example of a QNPPX circuit.
FIG. 9 is a diagram illustrating an example of a mathematical expression of QNPPX.
FIG. 10 is a flowchart illustrating an example of procedures of quantum chemical calculation by the VQE.
FIG. 11 is the first half of a flowchart illustrating an example of procedures in a quantum circuit generation process.
FIG. 12 is the second half of a flowchart illustrating an example of procedures in a quantum circuit generation process.
FIG. 13 is a diagram illustrating an example of an Ansatz circuit formed with rotation gate elements.
FIG. 14 is a diagram illustrating an example of quantum chemical calculation by the VQE.
FIG. 15 is a flowchart illustrating an example of base energy calculation procedures.
FIG. 16 is a graph illustrating a comparative example of a calculation result.
FIG. 17 is a table illustrating the relationship between the technique applied to an Ansatz circuit and the number of gates.
FIG. 18 is the first half of a flowchart illustrating an example of procedures for disposing rotation gate elements in an Ansatz circuit.
FIG. 19 is the second half of the flowchart illustrating an example of procedures for disposing rotation gate elements in an Ansatz circuit.
FIG. 20 is a diagram illustrating an example of an Ansatz circuit in which circuits of one-electron excitations and circuits of two-electron excitations coexist in the same column.

DESCRIPTION OF EMBODIMENTS

In the description below, embodiments will be explained with reference to the drawings. Note that each of the embodiments may be implemented in combination with a plurality of embodiments as long as there are no contradictions.

[First Embodiment]

A first embodiment is an information processing method for reducing the number of gate operations in a quantum circuit for performing quantum chemical calculation by the VQE.

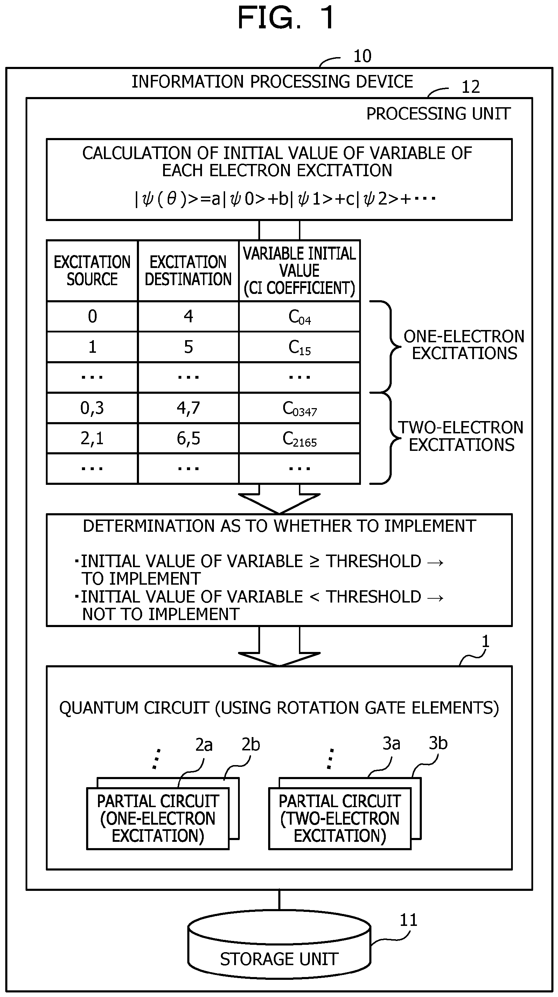

FIG. 1 is a diagram illustrating an example of the information processing method according to the first embodiment. In FIG. 1, an information processing device 10 for implementing the information processing method according to the first embodiment is illustrated. The information processing device 10 may implement the information processing method according to the first embodiment by executing an information processing program in which predetermined processing procedures are written, for example.

The information processing device 10 includes a storage unit 11 and a processing unit 12. The storage unit 11 is a memory or a storage device included in the information processing device 10, for example. The processing unit 12 is a processor or an arithmetic circuit included in the information processing device 10, for example.

The storage unit 11 stores information about the molecule to be calculated, a quantum circuit 1 for calculating the base energy of the molecule, and the like.

The processing unit 12 generates the quantum circuit 1 for performing quantum chemical calculation by the VQE using a quantum computer. For example, the processing unit 12 calculates the initial value of a variable corresponding to each electron excitation of a plurality of electron excitations for calculating the base energy of the molecule by the VQE. For example, the processing unit 12 calculates configuration interaction (CI) coefficients of the respective configuration state functions corresponding to a plurality of electron configurations included in wave functions (linear combinations of electron configurations) according to a CI method, using a classical quantum chemical calculation technique. The CI coefficients corresponding to the respective electron configurations are coefficients of a real number multiplied by a term indicating an electron excitation for the electron configuration. The processing unit 12 then sets the CI coefficient corresponding to each electron excitation as the initial value of the variable to be used in the circuit corresponding to the electron excitation.

Next, for each electron excitation of the plurality of electron excitations, the processing unit 12 determines whether to implement the corresponding circuit, based on whether the initial value of the corresponding variable is greater than a predetermined threshold. For example, for an electron excitation in which the absolute value of the initial value of the variable is greater than the threshold, the processing unit 12 determines to implement the circuit corresponding to the electron excitation. Further, when the initial value of the variable is equal to or smaller than the threshold, the processing unit 12 determines not to implement the circuit corresponding to the electron excitation.

The processing unit 12 then disposes, in the quantum circuit 1, partial circuits 2a, 2b, ..., 3a, 3b, ... that perform gate operations corresponding to the electron excitation for calculating the energy in accordance with the value of the variable, for each of the electron excitations for which it has been determined to implement the corresponding circuits among the plurality of electron excitations. For example, in a case where the electron excitation determined to be implemented are a one-electron excitation, the processing unit 12 disposes, in the quantum circuit 1, the partial circuits 2a, 2b,..., which perform operations of Givens rotation corresponding to the electron excitation. Furthermore, in a case where the electron excitation determined to be implemented is a two-electron excitation, the processing unit 12 disposes, in the quantum circuit 1, the partial circuits 3a, 3b,..., which perform operations of quantum-number-preserving-pair-exchange (QNPPX) corresponding to the electron excitation.

As described above, in a case where the initial value of the variable of an electron excitation is equal to or smaller than the threshold, implementation of the circuit corresponding to the electron excitation is prevented, to reduce the number of gate operations at the time of VQE calculation. For example, a CI coefficient is used as the initial value of a variable, it is expected that the value of an electron excitation to which a variable having a very small initial value is applied remains small even if optimization in VQE calculation is performed. The energy calculated by the circuit according to an electron excitation having a small variable value is minute compared with the energy calculated by circuits for other electron excitations. Therefore, even if implementation of the circuit corresponding to an electron excitation having a small variable value is prevented, the decrease in the calculation accuracy of the final base energy is small.

Moreover, a one-electron excitation gate operation is performed with a Givens rotation circuit, so that the number of gate operations can be further reduced without lowering the calculation accuracy. Likewise, a two-electron excitation gate operation is performed with QNPPX, so that the number of gate operations can be further reduced without lowering the calculation accuracy.

In VQE calculation, a gate operation and measurement of an electron excitation can be performed in parallel for each electron excitation. However, the number of quantum bits that can be used may not be sufficient to execute all electron excitations in parallel. In that case, it is possible to reduce the number of gate operations by generating the quantum circuit 1 so that more partial circuits can be executed in parallel without any overlap between the quantum bits of the excitation source and the excitation destination in the electron excitations to be simultaneously executed. Note that, in the quantum circuit 1, the circuits to be executed in parallel are disposed in the same column (positions overlapping in the vertical direction in the quantum circuit 1).

For example, it is assumed that the processing unit 12 separates a column in which partial circuits of one-electron excitations are disposed from a column in which partial circuits of two-electron excitations are disposed. Here, the partial circuits already disposed in a certain column in the quantum circuit 1 are set as first partial circuits. Further, the partial circuits to be disposed next are set as second partial circuits. In a case where the quantum bits of the excitation sources and the excitation destinations of the electron excitations corresponding to the second partial circuits do not overlap with any of the quantum bits of the excitation sources and the excitation destinations in the first partial circuits at this point of time, the processing unit 12 disposes the second partial circuits in the same column as the first partial circuits. Thus, the number of gate operations in the quantum circuit 1 is reduced.

Also, it is possible to further reduce the number of gate operations by mixing one-electron excitation partial circuits and two-electron excitation partial circuits in the same column. For example, the processing unit 12 disposes third partial circuits corresponding to two-electron excitations in a column in which circuits that can be simultaneously executed in the quantum circuit 1 are disposed. After that, the processing unit 12 disposes, in the same column as the third partial circuits, a plurality of fourth partial circuits that correspond to one-electron excitations and have the quantum bits of the excitation sources and the excitation destinations not overlapping with any of the quantum bits of the excitation sources and the excitation destinations in the third partial circuits. At that time, the processing unit 12 disposes the plurality of fourth partial circuits in the same column as the third partial circuits within a range in which the number of times of use of the same quantum bit as an excitation source or an excitation destination does not exceed a predetermined number among the plurality of fourth partial circuits. Thus, the quantum circuit 1 having the number of gate operations reduced is generated.

[Second Embodiment]

A second embodiment is a computer system that performs quantum chemical calculation by the VQE, using a quantum computer.

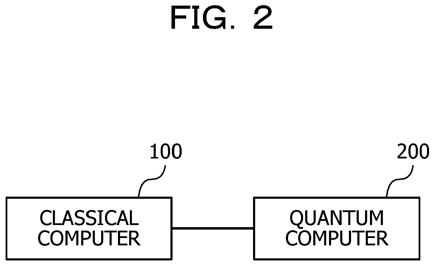

FIG. 2 is a diagram illustrating an example of a system configuration according to the second embodiment. In the second embodiment, a classical computer 100 and a quantum computer 200 are coupled. The classical computer 100 is a von Neumann computer, and performs processing such as generation of a quantum circuit and optimization of the parameters to be used in calculation of the quantum circuit. The quantum computer 200 is a computer that performs quantum chemical calculation by performing an operation based on a quantum gate on quantum bits. The quantum computer 200 performs quantum chemical calculation by a VQE algorithm in accordance with the quantum circuit and the parameters generated by the classical computer 100, and calculates an energy expectation value.

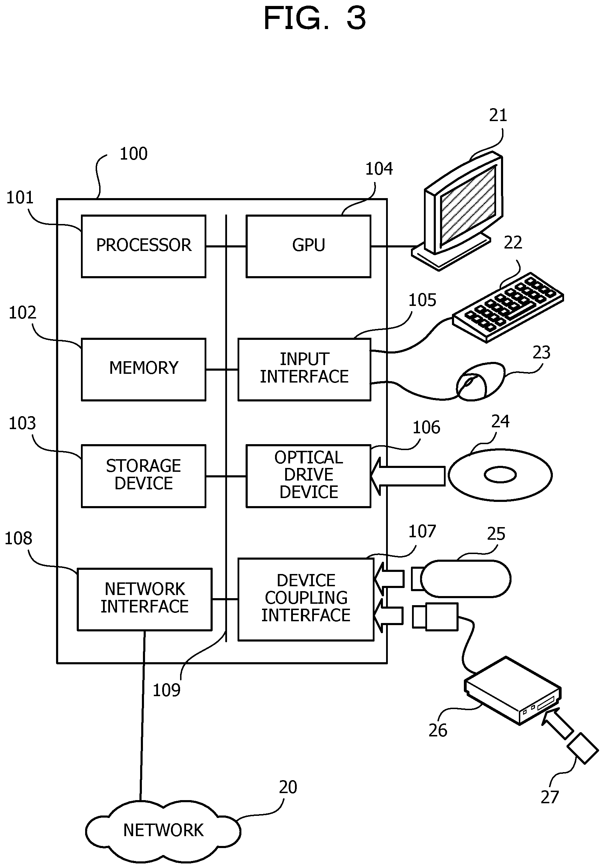

FIG. 3 is a diagram illustrating an example of the hardware of a classical computer. The entire device of the classical computer 100 is controlled by a processor 101. A memory 102 and a plurality of peripheral devices are coupled to the processor 101 via a bus 109. The processor 101 may be a multiprocessor. The processor 101 is a central processing unit (CPU), a micro processing unit (MPU), or a digital signal processor (DSP), for example. At least some of functions implemented by the processor 101 executing a program may be implemented by an electronic circuit such as an application specific integrated circuit (ASIC) or a programmable logic device (PLD).

The memory 102 is used as a main storage device of the classical computer 100. In the memory 102, at least some of the operating system (OS) programs and the application programs to be executed by the processor 101 are temporarily stored. Furthermore, in the memory 102, various kinds of data to be used in processing by the processor 101 are stored. As the memory 102, a volatile semiconductor storage device such as a random access memory (RAM) is used, for example.

Examples of the peripheral devices coupled to the bus 109 include a storage device 103, a graphics processing unit (GPU) 104, an input interface 105, an optical drive device 106, a device coupling interface 107, and a network interface 108.

The storage device 103 electrically or magnetically writes/reads data into/from a built-in recording medium. The storage device 103 is used as an auxiliary storage device of the classical computer 100. In the storage device 103, OS programs, application programs, and various kinds of data are stored. Note that, as the storage device 103, a hard disk drive (HDD) or a solid state drive (SSD) may be used, for example.

The GPU 104 is an arithmetic device that performs image processing, and is also called a graphics controller. A monitor 21 is coupled to the GPU 104. The GPU 104 causes the screen of the monitor 21 to display an image in accordance with an instruction from the processor 101. Examples of the monitor 21 include a display device using an organic electro luminescence (EL), a liquid crystal display device, and the like.

A keyboard 22 and a mouse 23 are coupled to the input interface 105. The input interface 105 transmits signals sent from the keyboard 22 and the mouse 23 to the processor 101. Note that the mouse 23 is an example of a pointing device, and some other pointing device may also be used. Examples of other pointing devices include a touch panel, a tablet, a touch pad, a track ball, and the like.

The optical drive device 106 uses laser light or the like, to read data recorded in an optical disk 24, or write data into the optical disk 24. The optical disk 24 is a portable recording medium in which data is recorded so that the data can be read through reflection of light. Examples of the optical disk 24 include a digital versatile disc (DVD), a DVD-RAM, a compact disc read only memory (CD-ROM), a CD-recordable (R)/rewritable (RW), and the like.

The device coupling interface 107 is a communication interface for coupling peripheral devices to the classical computer 100. For example, a memory device 25 and a memory reader/writer 26 may be coupled to the device coupling interface 107. The memory device 25 is a recording medium equipped with a function to communicate with the device coupling interface 107. The memory reader/writer 26 is a device that writes data into a memory card 27 or reads data from the memory card 27. The memory card 27 is a card-type recording medium.

The network interface 108 is coupled to the quantum computer 200. The network interface 108 exchanges data with the quantum computer 200.

The classical computer 100 may implement processing functions of the second embodiment with the hardware as described above. Note that the information processing device 10 described in the first embodiment can be formed with hardware similar to that of the classical computer 100 illustrated in FIG. 3.

The classical computer 100 implements the processing functions of the second embodiment by executing a program recorded in a computer-readable recording medium, for example. The programs in which the contents of the processes to be performed by the classical computer 100 are written may be recorded in various recording media. For example, the programs to be executed by the classical computer 100 may be stored in the storage device 103. The processor 101 loads at least one of the programs in the storage device 103 into the memory 102, and executes the program. Also, the programs to be executed by the classical computer 100 may be recorded in a portable recording medium such as the optical disk 24, the memory device 25, or the memory card 27. A program stored in the portable recording medium may be executed after being installed into the storage device 103 under the control of the processor 101, for example. Also, the processor 101 may read a program directly from the portable recording medium, to execute the program.

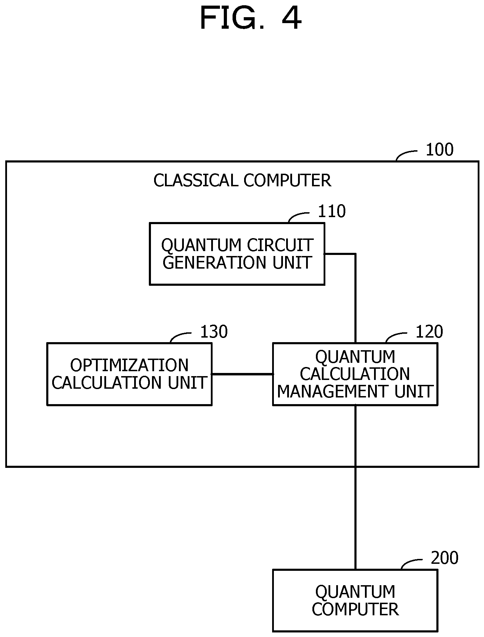

With the hardware illustrated in FIG. 4, the classical computer 100 may perform quantum chemical calculation by the VQE, in cooperation with the quantum computer 200.

FIG. 4 is a block diagram illustrating an example of functions of a classical computer for quantum chemical calculation by the VQE. The classical computer 100 includes a quantum circuit generation unit 110, a quantum calculation management unit 120, and an optimization calculation unit 130.

The quantum circuit generation unit 110 generates a quantum circuit for calculating energy of a quantum many-body system such as a molecule. For example, the quantum circuit generation unit 110 generates a quantum circuit based on a VQE algorithm that realizes an electron excitation of UCCSD Ansatz by fewer quantum operations. The quantum circuit generation unit 110 transmits the generated quantum circuit to the quantum calculation management unit 120.

The quantum calculation management unit 120 instructs the quantum computer 200 to perform energy calculation based on the generated quantum circuit. For example, the quantum calculation management unit 120 sets a plurality of parameters θ related to a gate operation at a quantum gate in the quantum circuit. The quantum calculation management unit 120 sets initial values to the values of the plurality of parameters θ before the first quantum calculation. The quantum calculation management unit 120 acquires, from the quantum computer 200, a result of the energy calculation based on the quantum circuit parameterized with the plurality of parameters θ. Having acquired the result of the energy calculation, the quantum calculation management unit 120 determines whether the energy has converged. When the energy has not converged, the quantum calculation management unit 120 instructs the optimization calculation unit 130 to optimize the parameters.

The optimization calculation unit 130 updates, for each quantum calculation, all or some of the values of the plurality of parameters θ in a direction in which the energy value decreases. When the optimization calculation comes to an end, the optimization calculation unit 130 notifies the quantum calculation management unit 120 of the updated values of the plurality of parameters θ.

Note that the function of each element of the classical computer 100 illustrated in FIG. 4 may be implemented by causing a computer to execute the program module corresponding to the element, for example.

Next, a method for calculating the base energy of a molecule by the VQE is described. The energy E to be obtained in calculating the base energy of a molecule by the VQE is expressed as in the following Expression (1).

[Math. 1]

$$E = \langle H \rangle = \frac{\langle \varphi(\vec{\theta}) | H | \varphi(\vec{\theta}) \rangle}{\varphi(\vec{\theta}) | \varphi(\vec{\theta})} \qquad (1)$$

φ represents a quantum state. H represents a Hamiltonian. The Hamiltonian is a function of an intermolecular distance R. θ represents the rotation angle to be used as the optimization variable. The base energy is the lowest energy. Therefore, when Expression (1) is repeatedly calculated by varying the rotation angle θ, the lowest energy among the energies obtained by a plurality of times of calculation is a base energy $E_0$. An expression expressing the base energy $E_0$ is as follows.

[Math. 2]

$$\frac{\langle \varphi(\vec{\theta}) | H | \varphi(\vec{\theta}) \rangle}{\varphi(\vec{\theta}) | \varphi(\vec{\theta})} \geq E_0 \qquad (2)$$

In actual calculation, the Hamiltonian is decomposed into a sum form ($H = H_1 + H_2 + ...$), and each Hamiltonian obtained by the decomposition is calculated. In other words, the quantum circuit generation unit 110 generates a quantum circuit for performing quantum chemical calculation by the VQE for each Hamiltonian obtained by the decomposition.

A quantum circuit of the VQE includes a circuit called Ansatz. Ansatz occupies most of the VQE quantum circuit.

Ansatz is a circuit for expressing a wave function ψ(θ) parameterized with a variable in a quantum circuit.

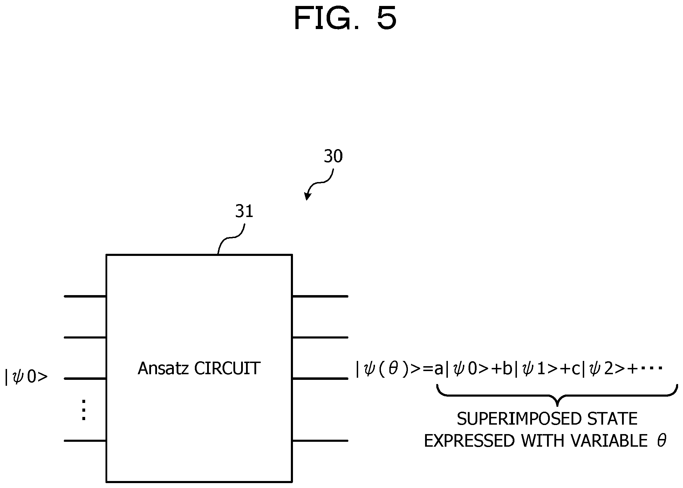

FIG. 5 is a diagram for explaining an Ansatz circuit. A wave function expressed by an Ansatz circuit 31 included in a quantum circuit 30 is a superimposed state expressed with a variable θ according to the CI method. The superimposed state is expressed by the following Expression (3) .

$$|\psi(\theta)>=a|\psi 0>+b|\psi 1>+c|\psi 2>+... \quad (3)$$

Each term on the right side of Expression (3) corresponds to an electron excitation for obtaining a predetermined electron orbital. a, b, and c are coefficients for each electron excitation. The quantum circuit 30 includes a basis conversion circuit, a Z-axis measurement circuit, and the like, in addition to the Ansatz circuit 31.

There is Unitary Coupled Cluster Singles and Doubles (UCCSD) as a representative Ansatz for achieving high accuracy. The UCCSD can obtain a calculation result with a higher accuracy than Coupled Cluster Singles and Doubles (CCSD) of classical quantum chemical calculation in an ideal state (when a hard error is small enough). On the other hand, even if the UCCSD is a problem of about 20 quantum bits, tens of thousands to hundreds of thousands of gates are required, and it is practically difficult to implement the UCCSD in a NISQ.

Therefore, the quantum circuit generation unit 110 generates a quantum circuit for realizing the VQE by expressing a one-electron excitation or a two-electron excitation using a rotation gate element (a Givens rotation circuit, or a QNPPX circuit). Thus, a calculation accuracy equivalent to that of the UCCSD can be achieved, and the number of gates can be reduced.

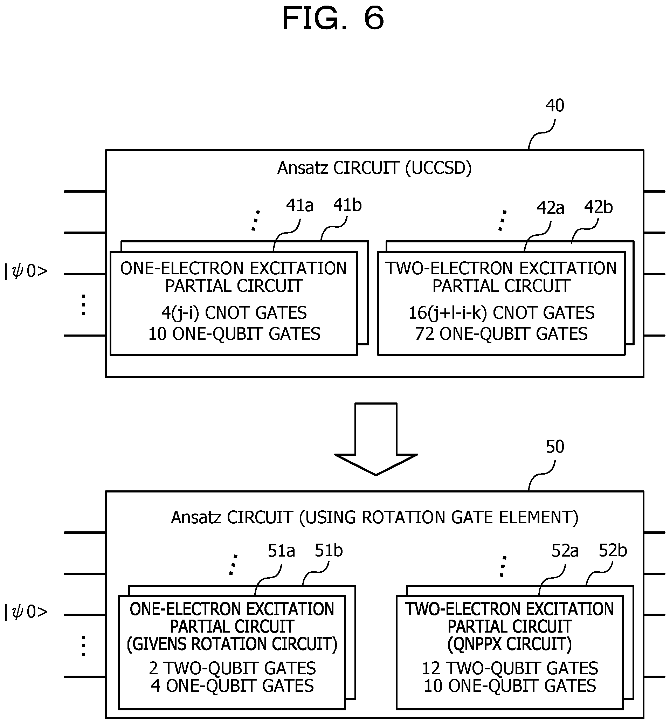

FIG. 6 is a diagram illustrating an example of a reduction in the number of gates with rotation gate elements. An Ansatz circuit 40 according to the UCCSD includes a plurality of one-electron excitation partial circuits 41a, 41b,..., and a plurality of two-electron excitation partial circuits 42a, 42b,....

Here, it is assumed that the one-electron excitation partial circuits 41a, 41b,... express one-electron excitations from the i-th quantum bit to the j-th quantum bit. In this case, for each one excitation, 4(j-i) CNOT gates and ten one-qubit gates are used in the one-electron excitation partial circuits 41a, 41b,... (i and j being integers of 0 or greater) . The CNOT gates are two-qubit gates that perform bit inversion on the target quantum bits when the control quantum bits are |1>.

Meanwhile, it is assumed that the two-electron excitation partial circuits 42a, 42b,... express two-electron excitations from the (i, j)-th quantum bit to the (k, l)-th quantum bit. In this case, for each one excitation, 16(j+l-i-k) CNOT gates and 72 one-qubit gates are used in the two-electron excitation partial circuits 42a, 42b,... (i, j, k, and l being integers of 0 or greater).

An Ansatz circuit 50 using a rotation gate element also includes a plurality of one-electron excitation partial circuits 51a, 51b,..., and a plurality of two-electron excitation partial circuits 52a, 52b,.... The one-electron excitation partial circuits 51a, 51b,... are circuits that express one-electron excitations with Givens rotation. For each one excitation, two two-qubit gates and four one-qubit gates are used in the one-electron excitation partial circuits 51a, 51b,.... Meanwhile, the two-electron excitation partial circuits 52a, 52b,... are circuits that express two-electron excitations with QNPPX. For each one excitation, twelve two-qubit gates and ten one-qubit gates are used in the two-electron excitation partial circuits 52a, 52b,....

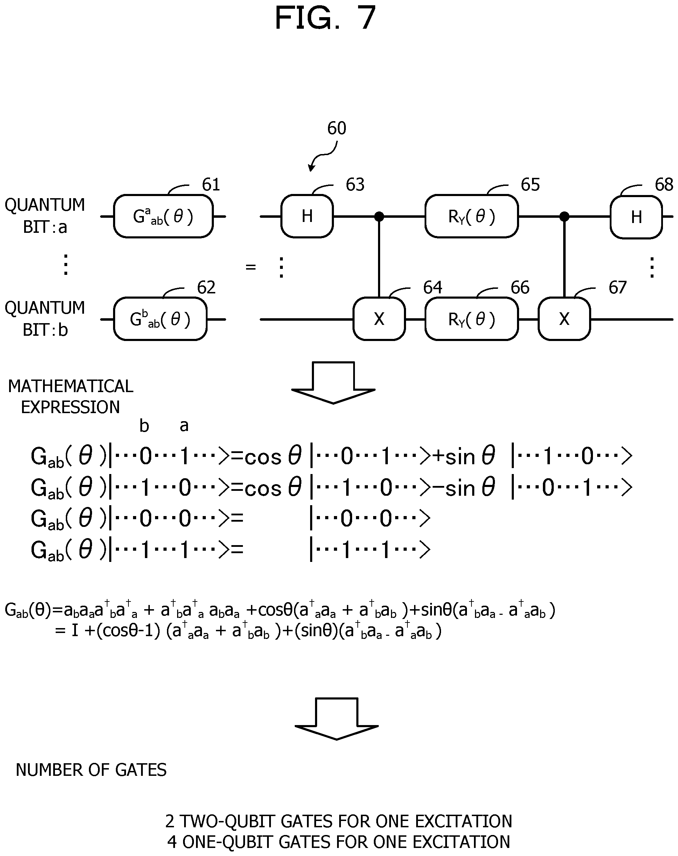

FIG. 7 is a diagram illustrating an example of a Givens rotation circuit. A Givens rotation circuit 60 in FIG. 7 illustrates an example of one-electron excitation that has the "a"-th quantum bit as the excitation source, and the "b"-th quantum bit as the excitation destination (a and b being integers of 0 or greater). Two circuit elements 61 and 62 indicating the Givens rotation circuit 60 are disposed on the horizontal lines corresponding to quantum bits to be subjected to one-electron excitations.

When the rotation angle is θ, the circuit element 61 corresponding to the "a"-th quantum bit that is the excitation source is expressed as "$G^{a}_{ab}(\theta)$". The circuit element 62 of the "b"-th quantum bit that is the excitation destination is expressed as "$G^{b}_{ab}(\theta)$", with θ being the rotation angle. The set of the circuit element 61 and the circuit element 62 represents one Givens rotation circuit 60.

Where the Givens rotation circuit 60 is expressed with one-qubit gates and two-qubit gates, an Hadamard gate 63 is first disposed in the quantum bit that is the excitation source. Next, a CNOT gate 64 that has the control quantum bit as the excitation source and the target quantum bit as the excitation destination is provided. Next, rotation gates 65 and 66 around the Y-axis at the rotation angle θ are disposed in the quantum bit as the excitation source and the quantum bit as the excitation destination, respectively. Next, a CNOT gate 67 that has the control quantum bit as the excitation source and the target quantum bit as the excitation destination is provided. Lastly, an Hadamard gate 68 is disposed in the quantum bit that is the excitation source.

This Givens rotation is expressed by Expression (4) as illustrated below.

$$G_{ab}(\theta)|...0...1...\rangle = \cos\theta|...0...1...\rangle + \sin\theta|...1...0...\rangle$$

$$G_{ab}(\theta)|...1...0...\rangle = \cos\theta|...1...0...\rangle - \sin\theta|...0...1...\rangle$$

$$G_{ab}(\theta)|...0...0...\rangle = \quad |...0...0...\rangle$$

$$G_{ab}(\theta)|...1...1...\rangle = \quad |...1...1...\rangle \qquad (4)$$

Of the two values expressed as "$|...0...1...\rangle$" in Expression (4), the value on the left side corresponds to the quantum state of the quantum bit of the excitation destination (the "b"-th quantum bit), and the value on the right side corresponds to the quantum state of the quantum bit of the excitation source (the "a"-th quantum bit). Further, the Givens rotation can also be expressed as in the following Expression (5).

$$G_{ab}(\theta) = a_b a_a a^\dagger_b a^\dagger_a + a^\dagger_b a^\dagger_a a_b a_a + \cos\theta \quad (a^\dagger_a a_a + a^\dagger_b a_b) + \sin\theta(a^\dagger_b a_a - a^\dagger_a a_b) \quad = I + \quad (\cos\theta-1)(a^\dagger_a a_a + a^\dagger_b a_b) + (\sin\theta)(a^\dagger_b a_a - a^\dagger_a a_b) \qquad (5)$$

In Expression (5), I represents an identity operator. As illustrated in FIG. 7, the only two-qubit gates included in the Givens rotation circuit 60 are the two CNOT gates 64 and 67. Accordingly, the number of two-qubit gates provided for a one-electron excitation is two. Further, the one-qubit gates included in the Givens rotation circuit 60 are the two Hadamard gates 63 and 68 and the two rotation gates 65 and 66. Accordingly, the number of one-qubit gates provided for a one-electron excitation is four.

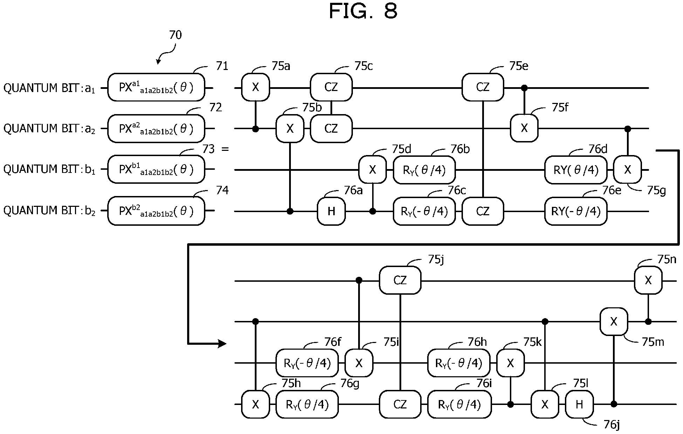

FIG. 8 is a diagram illustrating an example of a QNPPX circuit. In QNPPX, two quantum bits representing the orbit of the excitation source, and two quantum bits representing the orbit of the excitation destination are operated. A QNPPX circuit 70 illustrated in FIG. 8 illustrates an example of a two-quantum excitation in which the "$a_1$"-th and "$a_2$"-th quantum bits are excitation sources and the "$b_1$"-th and "$b_2$"-th quantum bits are excitation destinations ($a_1$, $a_2$, $b_1$, and $b_2$ being integers of 0 or greater). Four circuit elements 71 to 74 indicating the QNPPX circuit 70 are disposed on the horizontal lines corresponding to quantum bits to be subjected to two-electron excitations.

When the rotation angle is $\theta$, the circuit element 71 corresponding to the "$a_1$"-th quantum bit that is an excitation source is expressed as "$PX^{a1}_{a1a2b1b2}(\theta)$". The circuit element 72 corresponding to the "$a_2$"-th quantum bit that is an excitation source is expressed as "$PX^{a2}_{a1a2b1b2}(\theta)$". The circuit element 73 corresponding to the "$b_1$"-th quantum bit that is an excitation destination is expressed as "$PX^{b1}_{a1a2b1b2}(\theta)$". The circuit element 74 corresponding to the "$b_2$"-th quantum bit that is an excitation destination is expressed as "$PX^{b2}_{a1a2b1b2}(\theta)$".

Where the QNPPX circuit 70 is expressed with one-qubit gates and two-qubit gates, a CNOT gate 75a that has the "$a_2$"-th quantum bit as the control quantum bit and the "$a_1$"-th quantum bit as the target quantum bit is first provided. Next, a CNOT gate 75b that has the "$b_2$"-th quantum bit as the control quantum bit and the "$b_2$"-th quantum bit as the target quantum bit is provided. Next, a CZ gate 75c for the "$a_1$"-th quantum bit and the "$a_2$"-th quantum bit is provided, and an Hadamard gate 76a is disposed in the "$b_2$"-th quantum bit. Next, a CNOT gate 75d that has the "$b_2$"-th quantum bit as the control quantum bit and the "$b_1$"-th quantum bit as the target quantum bit is provided. Next, a rotation gate 76b around the Y-axis at the rotation angle $\theta$ is disposed in the "$b_1$"-th quantum bit, and a rotation gate 76c around the Y-axis at a rotation angle $-\theta$ is disposed in the "$b_2$"-th quantum bit.

Next, a CZ gate 75e for the "$a_1$"-th quantum bit and the "$b_2$"-th quantum bit is provided. Next, a CNOT gate 75f that has the "$a_1$"-th quantum bit as the control quantum bit and the "$a_2$"-th quantum bit as the target quantum bit is provided. Next, a rotation gate 76d around the Y-axis at the rotation angle $\theta$ is disposed in the "$b_1$"-th quantum bit, and a rotation gate 76e around the Y-axis at the rotation angle $-\theta$ is disposed in the "$b_2$"-th quantum bit. Next, a CNOT gate 75g that has the "$a_2$"-th quantum bit as the control quantum bit and the "$b_1$"-th quantum bit as the target quantum bit is provided.

Next, a CNOT gate 75h that has the "$a_2$"-th quantum bit as the control quantum bit and the "$b_2$"-th quantum bit as the target quantum bit is provided. Next, a rotation gate 76f around the Y-axis at the rotation angle $-\theta$ is disposed in the "$b_1$"-th quantum bit, and a rotation gate 76g around the Y-axis at the rotation angle $\theta$ is disposed in the "$b_2$"-th quantum bit. Next, a CNOT gate 75i that has the "$a_1$"-th quantum bit as the control quantum bit and the "$b_1$"-th quantum bit as the target quantum bit is provided. Next, a CZ gate 75j for the "$a_1$"-th quantum bit and the "$b_2$"-th quantum bit is provided. Next, a rotation gate 76h around the Y-axis at the rotation angle $-\theta$ is disposed in the "$b_1$"-th quantum bit, and a rotation gate 76i around the Y-axis at the rotation angle $\theta$ is disposed in the "$b_2$"-th quantum bit.

Next, a CNOT gate 75k that has the "$b_2$"-th quantum bit as the control quantum bit and the "$b_1$"-th quantum bit as the target quantum bit is provided. Next, a CNOT gate 75l that has the "$a_2$"-th quantum bit as the control quantum bit and the "$b_2$"-th quantum bit as the target quantum bit is provided. Next, an Hadamard gate 76j is disposed in the "$b_2$"-th quantum bit. Next, a CNOT gate 75m that has the "$b_2$"-th quantum bit as the control quantum bit and the "$a_2$"-th quantum bit as the target quantum bit is provided. Next, a CNOT gate 75n that has the "$a_2$"-th quantum bit as the control quantum bit and the "$a_1$"-th

quantum bit as the target quantum bit is provided.

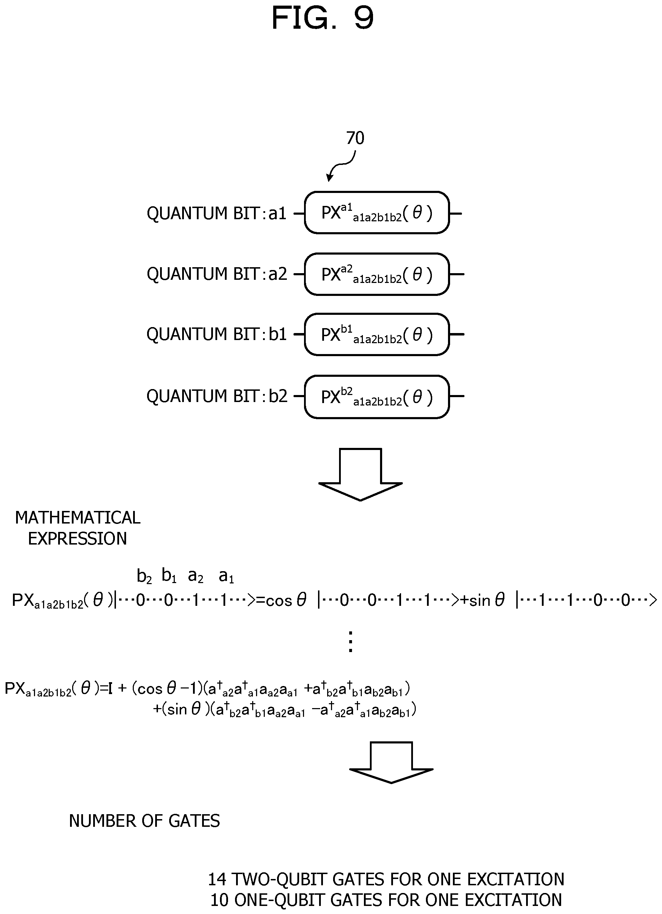

FIG. 9 is a diagram illustrating an example of a mathematical expression of QNPPX. For example, the QNPPX in a case where the states of the "$a_1$"-th and "$a_2$"-th quantum bits as excitation sources are both "1", and the states of the "$b_1$"-th and "$b_2$"-th quantum bits as excitation destinations are both "0" is expressed by the following Expression (6).

$$PX_{a1a2b1b2}(\theta)|...0...0...1...1...\rangle = \cos\theta|...0...0...1...\ 1...\rangle + \sin\theta|...1...1...0...0...\rangle... \tag{6}$$

The QNPPX operation for all states of each quantum bit is expressed by the following equation (7).

$$PX_{a1a2b1b2}(\theta) = I + (\cos\theta-1)(d^\dagger_{a2}a^\dagger_{a1}a_{a2}a_{a1} + a^\dagger_{b2}a^\dagger_{b1}a_{b2}a_{b1}) + (\sin\theta)(a^\dagger_{b2}a^\dagger_{b1}a_{a2}a_{a1} - a^\dagger_{a2}a^\dagger_{a1}a_{b2}a_{b1}) \tag{7}$$

The two-qubit gates of the QNPPX circuit 70 illustrated in FIG. 8 are the eleven CNOT gates 75a, 75b, 75d, 75f to 75i, and 75k to 75n, and the three CZ gates 75c, 75e, and 75j. Accordingly, the number of two-qubit gates provided for one two-electron excitation is 14. The one-qubit gates of the QNPPX circuit 70 are the two Hadamard gates 76a and 76j and the eight rotation gates 76b to 76i. Accordingly, the number of one-qubit gates provided for one two-electron excitation is ten.

As the one-electron excitations and the two-electron excitations are implemented with rotation gate elements in this manner, the number of gate operations can be reduced. Moreover, if the number of electron excitations implementing quantum circuits is small, the number of gate operations can be further reduced. In a case where the rotation angle $\theta$ in the rotation gate elements that realize one-electron excitations and two-electron excitations is small enough, the influence of the excitations on the wave function is very small. That is, the quantum circuits of a rotation gate element having a sufficiently small rotation angle $\theta$ has no significant influence on a calculation result even if the quantum circuit is not implemented. Therefore, the quantum circuit generation unit 110 prevents implementation of rotation gate elements to which a rotation angle $\theta$ equal to or smaller than the threshold is applied. Thus, the number of gate operations is reduced.

In the description below, a base energy calculation process by the VQE using a quantum circuit in which the number of gate operations is reduced will be described in detail.

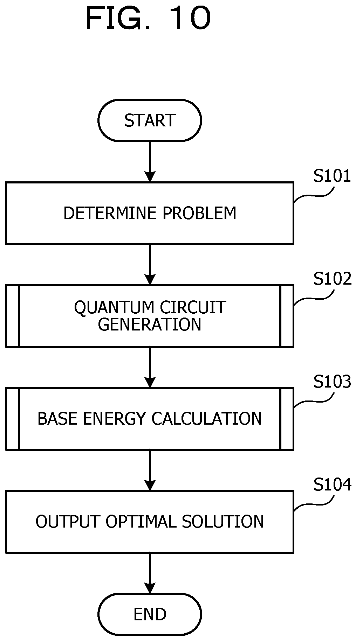

FIG. 10 is a flowchart illustrating an example of procedures of quantum chemical calculation by the VQE. In the description below, the processes illustrated in FIG. 10 will be explained in order of step number.

[Step S101] The quantum circuit generation unit 110 determines a problem to be calculated, based on an input from the user. For example, the quantum circuit generation unit 110 determines a molecular type, an atomic position, a basis function, an active space, or the like to be calculated. Also, the quantum circuit generation unit 110 determines association of the operators included in the Hamiltonian of the wave function with quantum bits, using Jordan-Wigner transformation, for example.

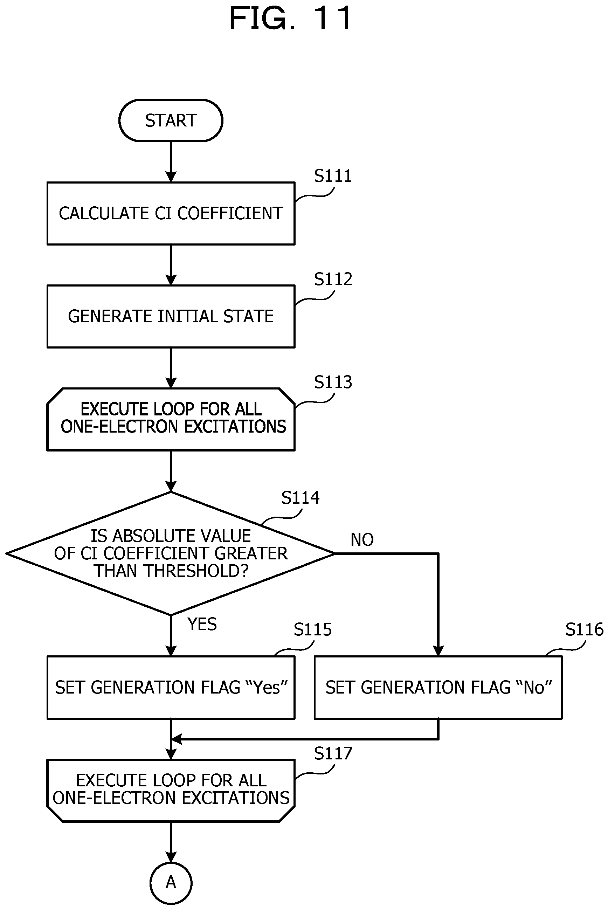

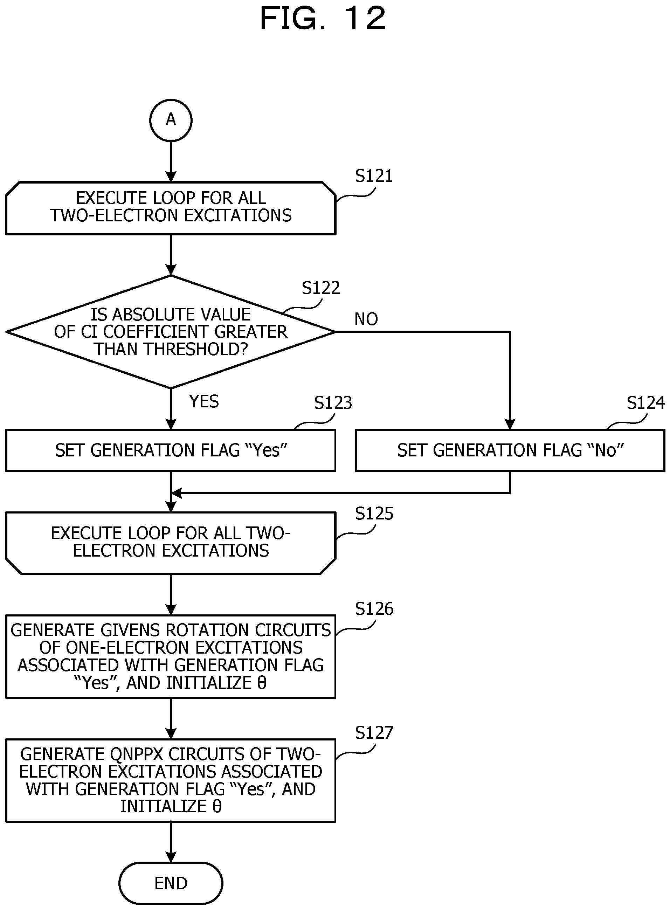

[Step S102] The quantum circuit generation unit 110 generates a quantum circuit that expresses electron excitations with rotation gate elements. The quantum circuit generation process will be described later in detail (see FIGS. 11 and 12).

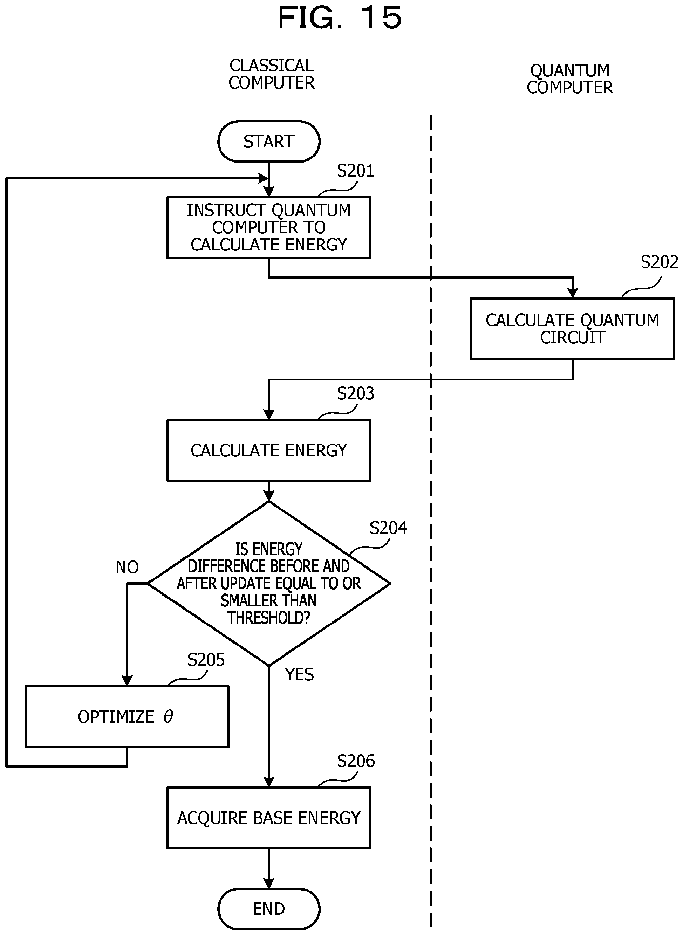

[Step S103] The quantum calculation management unit 120 controls the quantum computer 200 to calculate base energy. The VQE is used in calculating the base energy, and the optimization calculation unit 130 optimizes the optimization variable $\theta$. The base energy calculation process will be described later in detail (see FIG. 15).

[Step S104] The quantum calculation management unit 120 outputs a finally obtained optimal solution (a state in which energy has the minimum value).

Through such procedures, quantum chemical calculation by the VQE is performed. In the description below, each of the processes in quantum chemical calculation will be explained in detail.

FIG. 11 is the first half of a flowchart illustrating an example of procedures in the quantum circuit generation process. In the description below, the processes illustrated in FIG. 11 will be explained in order of step number.

[Step S111] The quantum circuit generation unit 110 calculates CI coefficients by a classical quantum mechanics calculation technique. Thus, CI coefficients corresponding to the respective electron excitations incorporated into the quantum circuit are calculated.

For example, the quantum circuit generation unit 110 calculates CI coefficients by the Configuration Interaction Singles and Doubles (CISD) method. CISD is a calculation in which a wave function as in Expression (8) below is created, and coefficients $C_{ab}$ and $C_{a1a2b1b2}$ are variably calculated so that the energy E expressed in Expression (9) becomes smaller.

$$|\psi\rangle = |\psi_{HF}\rangle + \Sigma C_{ab}a^\dagger_b a_a |\psi_{HF}\rangle + \Sigma C_{a1a2b1b2}a^\dagger_{a2}a^\dagger_{a1}a_{b2}a_{b1}|\psi_{HF}\rangle \tag{8}$$

$$E = \langle\psi|H|\psi\rangle \tag{9}$$

$C_{ab}$ and $C_{a1a2b1b2}$ are the CI coefficients. $C_{ab}$ is a CI coefficient corresponding to a one-electron excitation in which the orbit corresponding to the "a"-th quantum bit is an excitation source, and the orbit corresponding to the "b"-th quantum bit is an excitation destination. $C_{a1a2b1b2}$ is a CI coefficient corresponding to a two-electron excitation in which orbits corresponding to the "$a_1$"-th quantum bit and the "$a_2$"-th quantum bit are excitation sources, and orbits corresponding to the "$b_1$"-th quantum bit and the "$b_2$"-th quantum bit are excitation destinations. Meanwhile, "$a^\dagger_b a_a |\psi_{HF}\rangle$" and "$a^\dagger_{a2} a^\dagger_{a1} a_{b2} a_{b1} |\psi_{HF}\rangle$" represent the corresponding excitation-destination calculation bases (such as $|1100\rangle$, for example).

The calculated CI coefficients are used as the initial values of variables (the rotation angle $\theta$) to be applied to the rotation gate elements in the corresponding excitation (a one-electron excitation or a two-electron excitation) . If the CI coefficient ($C_{ab}$ or $C_{a1a2b1b2Cab}$) is small enough, it is possible to infer that the calculation basis corresponding to the CI coefficient is unnecessary in the wave function to be obtained after optimization by the VQE. Therefore, the quantum circuit generation unit 110 does not implement, in the quantum circuit in the VQE calculation, a circuit that calculates a calculation basis having a CI coefficient with an absolute value equal to or smaller than a threshold.

If the absolute value of the CI coefficient ($C_{ab}$ or $C_{a1a2b1b2}$) is greater than the threshold, the quantum circuit generation unit 110 uses the CI coefficient as the initial value of the variable for the corresponding circuit. The value of the variable is optimized by the optimization calculation unit 130 in the VQE calculation process.

[Step S112] The quantum circuit generation unit 110 generates an initial state of the wave function. For example, the quantum circuit generation unit 110 uses a Hartree Fock (HF) state as the initial state.

[Step S113] The quantum circuit generation unit 110 repeats the processes in steps S114 to S116 for all one-electron excitations.

[Step S114] The quantum circuit generation unit 110 determines whether the absolute value of the CI coefficient corresponding to the target one-electron excitation is greater than a predetermined threshold. If the absolute value of the CI coefficient is greater than the threshold, the quantum circuit generation unit 110 moves the process on to step S115. Furthermore, if the absolute value of the CI coefficient is not greater than the threshold, the quantum circuit generation unit 110 moves the process on to step S116.

[Step S115] The quantum circuit generation unit 110 sets a generation flag "Yes" associated with the target one-electron excitation. After that, the quantum circuit generation unit 110 moves the process on to step S117.

[Step S116] The quantum circuit generation unit 110 sets a generation flag "No" associated with the target one-electron excitation.

[Step S117] When the processes in steps S114 to S116 for all one-electron excitations are completed, the quantum circuit generation unit 110 moves the process on to step S121 (see FIG. 12).

FIG. 12 is the second half of the flowchart illustrating an example of procedures in the quantum circuit generation process. In the description below, the processes illustrated in FIG. 12 will be explained in order of step number.

[Step S121] The quantum circuit generation unit 110 repeats the processes in steps S121 to S124 for all two-electron excitations.

[Step S122] The quantum circuit generation unit 110 determines whether the absolute value of the CI coefficient corresponding to the target two-electron excitation is greater than a predetermined threshold. If the absolute value of the CI coefficient is greater than the threshold, the quantum circuit generation unit 110 moves the process on to step S123. Furthermore, if the absolute value of the CI coefficient is not greater than the threshold, the quantum circuit generation unit 110 moves the process on to step S124.

[Step S123] The quantum circuit generation unit 110 sets a generation flag "Yes" associated with the target two-electron excitation. After that, the quantum circuit generation unit 110 moves the process on to step S125.

[Step S124] The quantum circuit generation unit 110 sets a generation flag "No" associated with the target two-electron excitation.

[Step S125] When the processes in steps S122 to S124 for all two-electron excitations are completed, the quantum circuit generation unit 110 moves the process on to step S126.

[Step S126] The quantum circuit generation unit 110 generates Givens rotation circuits corresponding to the one-electron excitations associated with the generation flag "Yes". The quantum circuit generation unit 110 then disposes the generated Givens rotation circuits in the Ansatz circuit 31. Also, the quantum circuit generation unit 110 initializes the rotation angle $\theta$ of the disposed Givens rotation circuits to the value of the corresponding CI coefficient. For example, "$C_{ab}$" is set as the initial value of $\theta$ in the Givens rotation circuit "$G_{ab}(\theta)$" that has the "a"-th quantum bit as the excitation source and the "b"-th quantum bit as the excitation destination.

[Step S127] The quantum circuit generation unit 110 generates QNPPX circuits corresponding to the two-electron excitations associated with the generation flag "Yes". The quantum circuit generation unit 110 then disposes the generated QNPPX circuits in the Ansatz circuit 31. Also, the quantum circuit generation unit 110 initializes the rotation angle $\theta$ of the disposed QNPPX circuits to the value of the corresponding CI coefficient. For example, "$C_{a1a2b1b2}$" is set as the initial value of $\theta$ in the QNPPX circuit "$PX_{a1a2b1b2}(\theta)$" that has the "$a_1$"-th and "$a_2$"-th quantum bits as excitation sources and the "$b_1$"-th and "$b_2$"-th quantum bits as excitation destinations.

As illustrated in FIGS. 11 and 12, the circuits corresponding to the calculation bases having CI coefficients equal to or smaller than the threshold are not implemented in the Ansatz circuit 31 in the VQE. Thus, the number of gate operations in the VQE calculation is reduced.

Note that it is possible to estimate that the value of the coefficient of the circuit corresponding to a one-electron excitation or a two-electron excitation after optimization by the VQE is close to the absolute value of the CI coefficient. Accordingly, by setting the CI coefficient as the VQE parameter initial value to the rotation angle of the corresponding excitation, it is possible to accelerate convergence of a VQE calculation result, and perform the processing more efficiently.

Further, if the value of the coefficient of the circuit corresponding to a one-electron excitation or a two-electron excitation after optimization in the VQE is close to the absolute value of the CI coefficient, exclusion of the circuits corresponding to the calculation bases having CI coefficients equal to or smaller than the threshold from the VQE hardly affect the VQE calculation result. In other words, degradation of calculation accuracy is prevented.

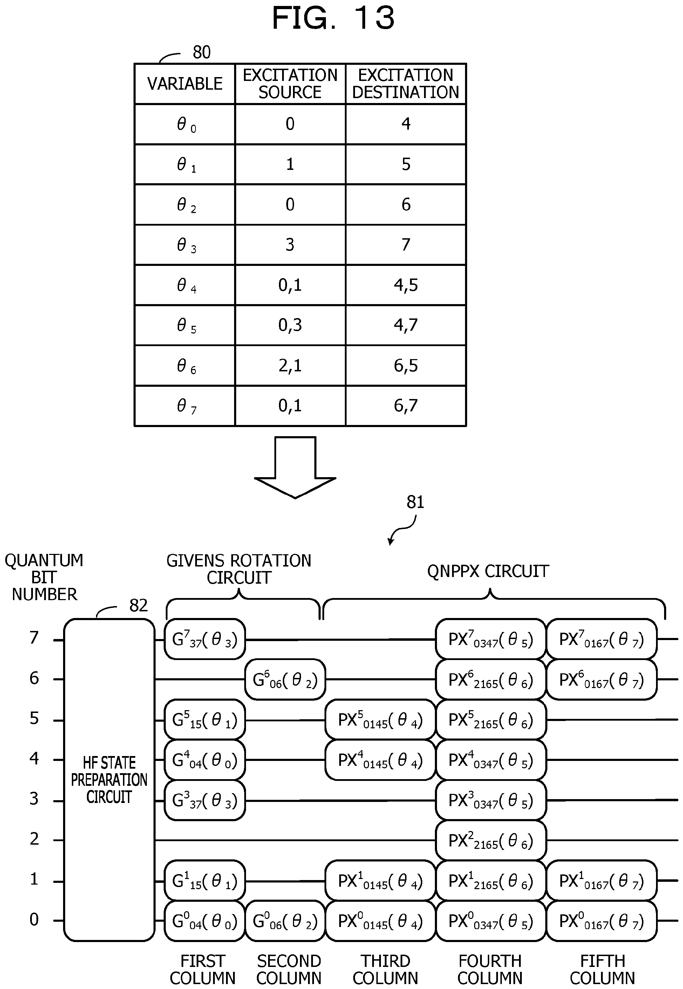

FIG. 13 is a diagram illustrating an example of an Ansatz circuit formed with rotation gate elements. For example, the quantum circuit generation unit 110 generates a generation table 80 indicating the one-electron excitations or the two-electron excitations associated with the generation flag "Yes". In the generation table 80, the quantum numbers of the excitation sources and the quantum numbers of the excitation destinations of the excitations to which variables are applied are set and associated with the variable names of the variables to be applied. In the case of a one-electron excitation, the excitation source and the excitation destination each have one quantum number. In the case of a two-electron excitation, the excitation source and the excitation destination each have two quantum numbers.

The quantum circuit generation unit 110 generates an Ansatz circuit 81 while referring to the generation table 80. In the Ansatz circuit 81, a HF state preparation circuit 82 for preparing a HF state is first disposed. Following the HF state preparation circuit 82, a plurality of Givens rotation circuits corresponding to one-electron excitations are provided.

As long as the quantum bits of excitation sources and excitation destinations do not overlap, the quantum circuit generation unit 110 disposes circuits in the first column (the column closer to the left side in the quantum circuit). For example, the quantum circuit generation unit 110 disposes the corresponding circuits in order from the top of the generation table 80. Note that the width of a column in which Givens rotation circuits are disposed is equal to the width of one Givens rotation circuit. Also, the width of a column in which QNPPX circuits are disposed is equal to the width of one QNPPX circuit.

For example, the quantum circuit generation unit 110 first disposes, in the first column, a Givens rotation circuit that has the quantum bit with a quantum bit number "0" as the excitation source, and the quantum bit with a quantum bit number "4" as the excitation destination. The quantum circuit generation unit 110 next disposes, in the same first column, a Givens rotation circuit that has the quantum bit with a quantum bit number "1" as the excitation source, and the quantum bit with a quantum bit number "5" as the excitation destination.

The quantum bit number of the excitation source of the Givens rotation circuit to be next disposed (the third row in the generation table 80) is "0". In the quantum bit with the quantum bit number "0", a circuit has already been disposed in the first column. Therefore, the quantum circuit generation unit 110 next disposes, in the second column, a Givens rotation circuit that has the quantum bit with the quantum bit number "0" as the excitation source, and the quantum bit with a quantum bit number "6" as the excitation destination.

Further, in the Givens rotation circuit to be disposed (the fourth row in the generation table 80), the quantum bit number of the excitation source is "3", and the quantum bit number of the excitation destination is "7". For the quantum bits corresponding to those quantum bit numbers, there is not a circuit disposed in the first column. Therefore, the quantum circuit generation unit 110 disposes, in the first column, a Givens rotation circuit that has the quantum bit with the quantum bit number "3" as the excitation source, and the quantum bit with the quantum bit number "7" as the excitation destination.

Likewise, the QNPPX circuits corresponding to two-electron excitations are disposed on the left side as long as there are no overlaps among the quantum bits. As a result, in the third column, QNPPX circuits that have the respective quantum bits with quantum bit numbers "0, 1" as excitation sources, and the respective quantum bits with quantum bit numbers "4, 5" as excitation destinations are disposed.

In the fourth column, QNPPX circuits that have the respective quantum bits with quantum bit numbers "0, 3" as excitation sources, and the respective quantum bits with quantum bit numbers "4, 7" as excitation destinations are disposed. Further, in the fourth column, QNPPX circuits that have the respective quantum bits with quantum bit numbers "2, 1" as excitation sources, and the respective quantum bits with quantum bit numbers "6, 5" as excitation destinations are disposed. In the fifth column, QNPPX circuits that have the respective quantum bits with the quantum bit numbers "0, 1" as excitation sources, and the respective quantum bits with quantum bit numbers "6, 7" as excitation destinations are disposed.

As the circuits are arranged in such a manner, the Ansatz circuit 81 having a small circuit depth is generated. Quantum chemical calculation by the VQE is performed by a quantum circuit including the Ansatz circuit 81 generated in such a manner. The quantum chemical calculation by the VQE is executed by a cooperative process between the quantum computer 200 and the classical computer 100.

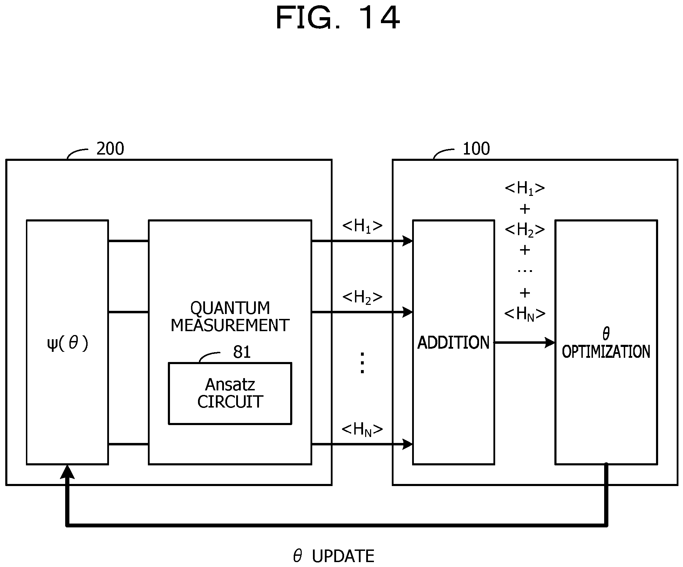

FIG. 14 is a diagram illustrating an example of quantum chemical calculation by the VQE. In quantum chemical calculation by the VQE, the quantum computer 200 performs quantum measurement based on the initial value of the parameters $\theta$(a set of variables $\theta_{ab}$ and $\theta_{a1a2b1b2}$ of the circuits corresponding to electron excitations). That is, the quantum computer 200 calculates the respective values of a plurality of divided Hamiltonians ($H_1$, $H_2$, ..., and $H_N$) in accordance with the quantum circuit. The quantum circuit that is used in the quantum measurement includes the Ansatz circuit 81 as illustrated in FIG. 13. The plurality of calculated Hamiltonians is added up by the classical computer 100, to turn into an expectation value of energy of the entire system. The optimization calculation unit 130 in the classical computer 100 then optimizes the parameters $\theta$ based the expectation value of energy. In other words, the optimization calculation unit 130 updates the parameters $\theta$ in a direction in which the expectation value of energy decreases. After the parameters $\theta$ are updated, the quantum computer 200 again calculates Hamiltonians based on the updated parameters $\theta$.

The calculation of Hamiltonians and the update of the parameters $\theta$ as described above are repeated until the base energy is obtained.

FIG. 15 is a flowchart illustrating an example of base energy calculation procedures. In the description below, the processes illustrated in FIG. 15 will be explained in order of step number.

[Step S201] The quantum calculation management unit 120 of the classical computer 100 instructs the quantum computer 200 to calculate energy with the quantum circuit for VQE calculation. At that time, the quantum calculation management unit 120 designates the parameters $\theta$ to be applied to the quantum circuit. CI coefficients are used as the initial values of the parameters $\theta$. After optimization of $\theta$, the values of the parameters $\theta$ updated by the optimization are designated as the values of variables.

[Step S202] The quantum computer 200 operates the quantum bits in accordance with the quantum circuit, and calculates the quantum circuit. The quantum computer 200 transmits the result of measurement for each quantum bit to the classical computer 100.

[Step S203] The quantum calculation management unit 120 of the classical computer 100 calculates energy from the results of measurement of the quantum bits.

[Step S204] The quantum calculation management unit 120 determines whether the energy difference before and after the update of the parameters $\theta$ is equal to or smaller than a predetermined threshold in the second and subsequent energy calculations. If the energy difference is equal to or smaller than the threshold, the quantum calculation management unit 120 moves the process on to step S206. Furthermore, if the energy difference is neither equal to nor smaller than the threshold, the quantum calculation management unit 120 moves the process on to step S205. Note that the quantum calculation management unit 120 invariably moves the process on to step S205 after the first energy calculation.

[Step S205] The optimization calculation unit 130 updates the parameters $\theta$ in a direction in which the expectation value of energy decreases. The quantum calculation management unit 120 acquires the updated values of the parameters $\theta$ from the optimization calculation unit 130, and moves the process on to step S201.

[Step S206] The quantum calculation management unit 120 acquires the minimum value of the repeatedly calculated energy as the base energy.

In this manner, a result of calculation of the base energy can be obtained. The accuracy of the obtained calculation result is equal to that by the UCCSD.

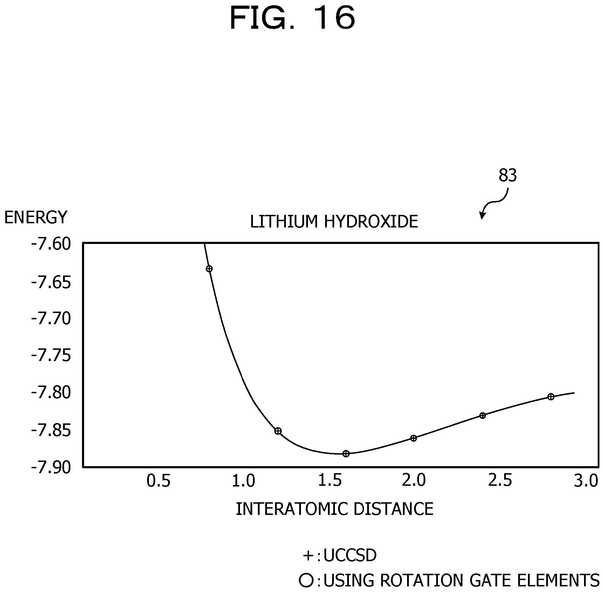

FIG. 16 is a graph illustrating a comparative example of a calculation result. In FIG. 16, the energy values for each interatomic distance in a case where the UCCSD is used and in a case where rotation gate elements are used are indicated. In a graph 83 illustrated in FIG. 16, the abscissa axis indicates interatomic distance, and the ordinate axis indicates energy. The energy values obtained by the UCCSD are indicated with "+" signs. Meanwhile, the energy values obtained by an Ansatz circuit using rotation gate elements are indicated with "o" signs. The threshold for determining whether to incorporate an excitation circuit into the Ansatz circuit using rotation gate elements is $10^{-20}$.

As illustrated in the graph 83, the energy values by the UCCSD and the energy values in a case where rotation gate elements are used are substantially the same values, and the "+" marks and the "o" marks overlap. That is, even if one-electron excitations and two-electron excitations are replaced with rotation gate elements, the calculation can be performed with an accuracy equal to that with the UCCSD.

Further, as rotation gate elements are used in the Ansatz circuit, the number of gates is greatly reduced compared with that with the UCCSD. Note that the number of gates in a case where rotation gate elements are used in the Ansatz circuit varies depending on the threshold for determining whether to incorporate an excitation circuit.

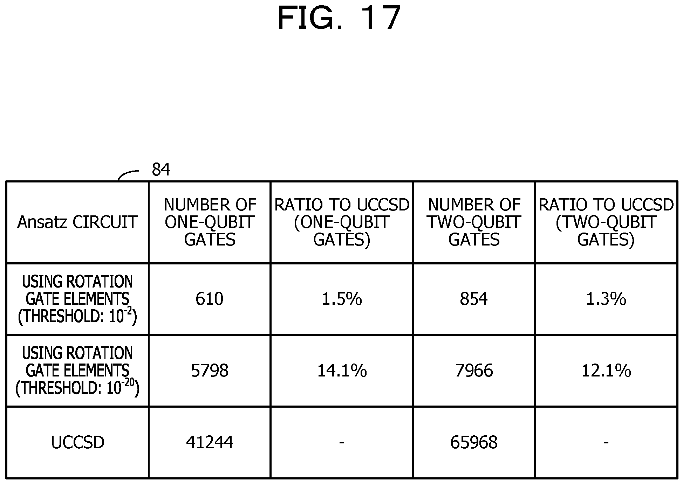

FIG. 17 is a table illustrating the relationship between the technique applied to an Ansatz circuit and the number of gates. FIG. 17 illustrates a gate number correspondence table 84 indicating the number of gates for each technique to be applied to an Ansatz circuit. In the gate number correspondence table 84, the number of one-qubit gates, the ratio of the number of one-qubit gates to the UCCSD, the number of two-qubit gates, and the ratio of the number of two-qubit gates to the UCCSD are indicated and associated with the names of the techniques to be applied to an Ansatz circuit.

In an Ansatz using rotation gate elements (threshold "$10^{-2}$"), the number of one-qubit gates is "610", and the number of two-qubit gates is "854". In an Ansatz using rotation gate elements (threshold "$10^{-20}$"), the number of one-qubit gates is "5798", and the number of two-qubit gates is "7966". In an Ansatz of the UCCSD, the number of one-qubit gates is "41244", and the number of two-qubit gates is "65968".

In the Ansatz using rotation gate elements (threshold "$10^{-2}$"), the number of one-qubit gates decreases to 1.5%, and the number of two-qubit gates decreases to 1.3%, compared with the UCCSD. In the Ansatz using rotation gate elements (threshold "$10^{-20}$"), the number of one-qubit gates decreases to 14.1%, and the number of two-qubit gates decreases to 12.1%, compared with the UCCSD.

As described above, even in a case where the threshold is set to "$10^{-20}$" with which a degree of accuracy similar to that with the UCCSD can be obtained, there is an effect of reducing the number of gates by 80% to 90% compared with the UCCSD. Furthermore, if the threshold is increased to about "$10^{-2}$" by allowing a decrease in accuracy, there is an effect of reducing the number of gates by 98% to 99% compared with the UCCSD. Accordingly, the threshold is made greater in accordance with the accuracy desired in quantum chemical calculation, for example, so that the speed of calculation can be increased.

In the description below, a specific example of molecular energy calculation will be explained. The object in question is $N_2$ (nitrogen molecules). The number of quantum bits to be used is 20 quantum bits. It is assumed that the base energy corresponding to the bond length (interatomic distance) is then calculated.

For CI coefficient calculation, classical quantum chemical calculation software is used. It is assumed that a threshold $\varepsilon$ is determined, CI coefficients $C_{ab}$ and $C_{a1a2b1b2}$ satisfying $\varepsilon < |C|$ are selected, and circuits of excitations corresponding to the selected CI coefficients are mounted in an Ansatz circuit. The initial state is "$\psi_{(HF)} = |$ 00000011111111111111>".

In the implementation of electron excitations, in the case of "$\varepsilon = 10^{-3}$", one-electron excitations corresponding to $C_{6,18}$ and $C_{7,19}$ are implemented in Givens rotation circuits. Here, a set including $C_{6,18}$ and $C_{7,19}$ is represented by $\tau1$, and the wave function till implementation of one-electron excitations is expressed by the following Expression (10).
[Math. 3]

$$\psi_{\left(\text{till implementation of one-electron excitations}\right)} = \prod_{\tau1} G_{ab}(C_{ab})\psi_{(HF)} \qquad (10)$$

In a manner similar to that for one-electron excitations, QNPPX circuits of two-electron excitations corresponding to the CI coefficients equal to or greater than the threshold $\varepsilon$ are implemented. Where a set of the CI coefficients exceeding the threshold $\varepsilon$ is represented by $\tau2$, the wave function is expressed by the following Expression (11).
[Math. 4]

$$\psi = \prod_{\tau2} P_{a1a2b1b2}(C_{a1a2b1b2}) \prod_{\tau1} G_{ab}(C_{ab})\psi_{(HF)} \qquad (11)$$

The base energy obtained by the optimization calculation unit 130 optimizing the parameters for the wave function is "-107.6453". This is a result sufficiently close to the exact solution "-107.6528" obtained by Full CI, which accurately solves the Schrödinger equation.

[Third Embodiment]

In a third embodiment, when rotation gate elements are disposed in an Ansatz circuit, circuits of one-electron excitations and circuits of two-electron excitations are allowed to coexist in one column having a width equal to the width of one QNPPX circuit. As Givens rotation circuits corresponding to one-electron excitations and QNPPX circuits corresponding to two-electron excitations are allowed to coexist in the same column, circuit placement that can be efficiently calculated becomes possible.

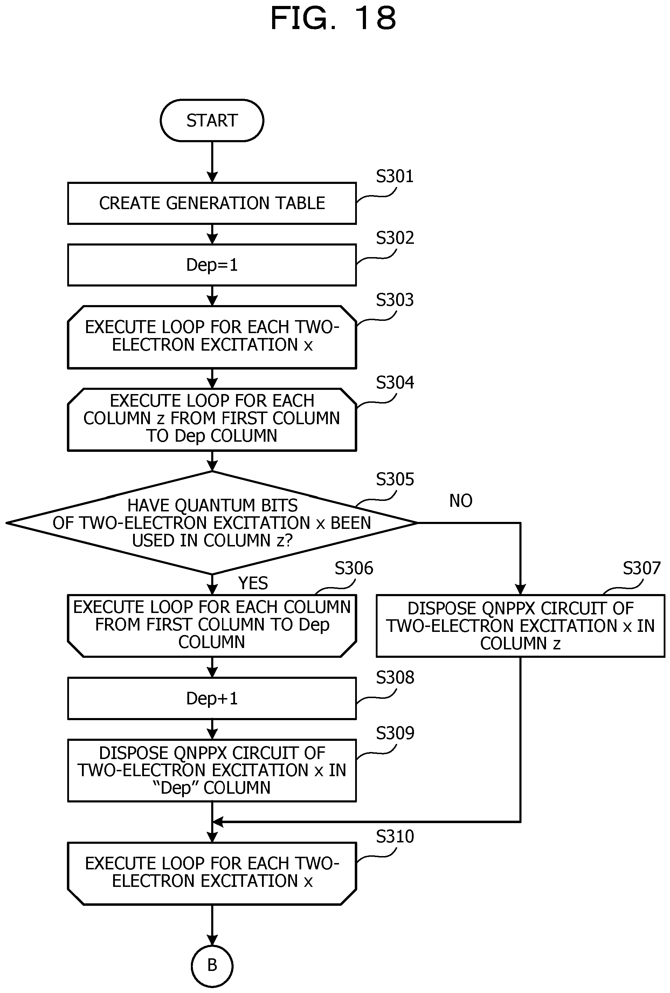

For example, the quantum circuit generation unit 110 performs processes illustrated in FIGS. 18 to 19 described below, instead of the processes in steps S126 and S127 (see FIG. 12) described in the second embodiment.

FIG. 18 is the first half of a flowchart illustrating an example of procedures for placing rotation gate elements in an Ansatz circuit. In the description below, the processes illustrated in FIG. 18 will be explained in order of step number.

[Step S301] The quantum circuit generation unit 110 creates a generation table. For example, the quantum circuit generation unit 110 registers, in the generation table, records corresponding to the respective one-electron excitations and two-electron excitations associated with the generation flag "Yes". In the registered record, the quantum bit numbers of the excitation sources and the excitation destinations are indicated and associated with the variables indicating rotation angles.

[Step S302] The quantum circuit generation unit 110 sets an initial value "1" to a variable "Dep" (Dep = 1) indicating the largest column number that can be a circuit placement position in the Ansatz circuit.

[Step S303] The quantum circuit generation unit 110 performs a process of determining a placement position for each two-electron excitation indicated in the generation table (steps S304 to S309). Here, the two-electron excitation for which the placement position is to be determined is represented by "x".

[Step S304] The quantum circuit generation unit 110 performs a placement possibility determination process (step S305) for each column from the first column to the Dep column. Here, the determination target column is a column z.

[Step S305] The quantum circuit generation unit 110 determines whether at least one quantum bit of an excitation source and an excitation destination of the two-electron excitation x in the column z has been used. If at least one quantum bit has been used, the quantum circuit generation unit 110 moves the process on to step S306. Furthermore, if all the quantum bits are unused, the quantum circuit generation unit 110 moves the process on to step S307.

[Step S306] In a case where the determination in step S305 is completed for all the columns from the first column to the Dep column, the quantum circuit generation unit 110 moves the process on to step S308.

[Step S307] The quantum circuit generation unit 110 disposes the QNPPX circuit corresponding to the two-electron excitation x to be subjected to the placement position determination in the column z that is the determination target in step S305. After that, the quantum circuit generation unit 110 moves the process on to step S310.

[Step S308] The quantum circuit generation unit 110 increments the variable "Dep" by 1 (Dep + 1).

[Step S309] The quantum circuit generation unit 110 disposes the QNPPX circuit corresponding to the two-electron excitation x to be subjected to the placement position determination in the Dep column after the increment.

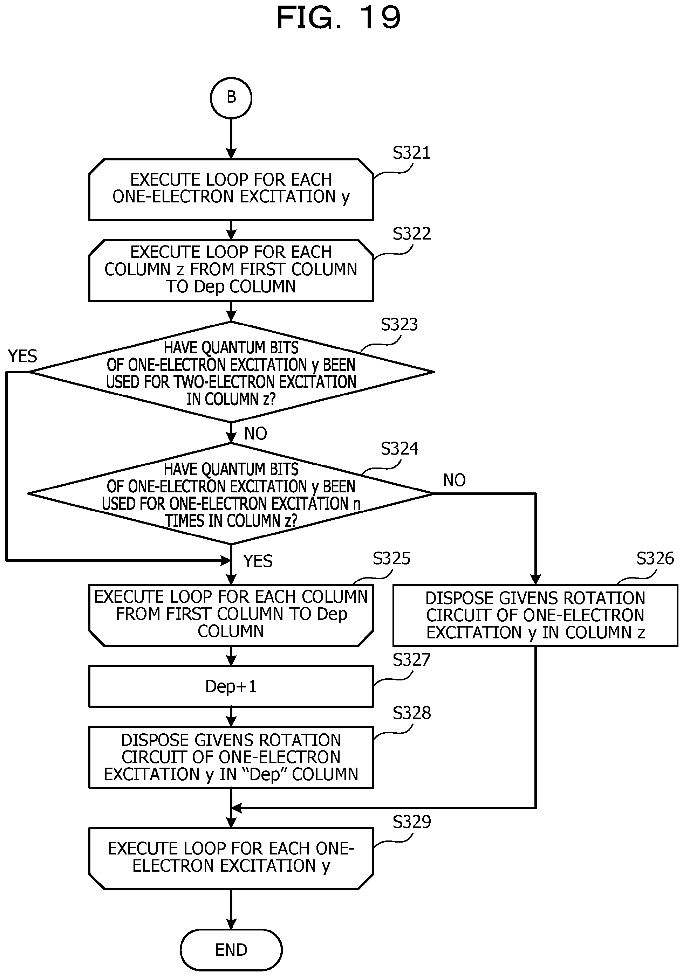

[Step S310] In a case where the processes in steps S304 to S309 have been performed for all two-electron excitations indicated in the generation table, the quantum circuit generation unit 110 moves the process on to step S321 (see FIG. 19).

FIG. 19 is the second half of the flowchart illustrating an example of procedures for placing rotation gate elements in an Ansatz circuit. In the description below, the processes illustrated in FIG. 19 will be explained in order of step number.

[Step S321] The quantum circuit generation unit 110 performs a process of determining a placement position for each one-electron excitation indicated in the generation table (steps S322 to S328). Here, the one-electron excitation for which the placement position is to be determined is represented by "y".

[Step S322] The quantum circuit generation unit 110 performs a determination process in steps S323 and S324 for each column from the first column to the Dep column. Here, the determination target column is the column z.

[Step S323] The quantum circuit generation unit 110 determines whether at least one quantum bit of the excitation source and the excitation destination of the one-electron excitation y in the column z has been used for a two-electron excitation. If at least one quantum bit has been used, the quantum circuit generation unit 110 moves the process on to step S325. Furthermore, if any of the quantum bits is unused, the quantum circuit generation unit 110 moves the process on to step S324.

[Step S324] The quantum circuit generation unit 110 determines whether at least one quantum bit of the excitation source and the excitation destination of the one-electron excitation y in the column z has been used n times for other one-electron excitations. n is a natural number indicating how many quantum operations for one-electron excitations can be implemented within the time of one quantum operation for one two-electron excitation. The value of n is determined by the process execution time for a one-qubit gate and the process execution time for a two-qubit gate. For example, n is a value of about "5".

In a case where at least one of the target quantum bits of the one-electron excitation y has been used n times in the column z, the quantum circuit generation unit 110 moves the process on to step S325. Furthermore, in a case where all the quantum bits have been used only up to n-1 times, the quantum circuit generation unit 110 moves the process on to step S326.

Note that, even in a case where any quantum bit of the target one-electron excitation y has been used only up to n-1 times, the quantum circuit generation unit 110 moves the process on to step S325 when the position in which the Givens rotation circuit for the one-electron excitation y is to be mounted is not found in the column z. For example, when the column z is divided into n subdivided columns, the Givens rotation circuits corresponding to one-electron excitations are disposed in the subdivided columns. If there is an unused subdivided column in which both the two quantum bits of the excitation source and the excitation destination of the one-electron excitation y to be mounted are unused, the one-electron excitation y can be mounted in the column z. If there are no subdivided columns in which both the two quantum bits of the excitation source and the excitation destination of the one-electron excitation y to be disposed are unused, the one-electron excitation y cannot be implemented in the column z, and therefore, the process is moved on to step S325.

[Step S325] In a case where the determination in steps S323 and S324 is completed for all the columns from the first column to the Dep column, the quantum circuit generation unit 110 moves the process on to step S327.

[Step S326] The quantum circuit generation unit 110 disposes the Givens rotation circuit corresponding to the one-electron excitation y to be subjected to the placement position determination in the column z that is the determination target in steps S323 and S324. After that, the quantum circuit generation unit 110 moves the process on to step S329.

[Step S327] The quantum circuit generation unit 110 increments the variable "Dep" by 1 (Dep + 1).

[Step S328] The quantum circuit generation unit 110 disposes the Givens rotation circuit corresponding to the one-electron excitation y to be subjected to the placement position determination in the Dep column after the increment.

[Step S329] In a case where the processes in steps S322 to S327 have been performed for all the one-electron excitations indicated in the generation table, the quantum circuit generation unit 110 ends the rotation gate element placement process.

In this manner, an Ansatz circuit in which Givens rotation circuits of one-electron excitations and QNPPX circuits of two-electron excitations coexist in the same column is generated. The generated Ansatz circuit efficiently utilizes each quantum bit, and reduces the depth of the circuit.

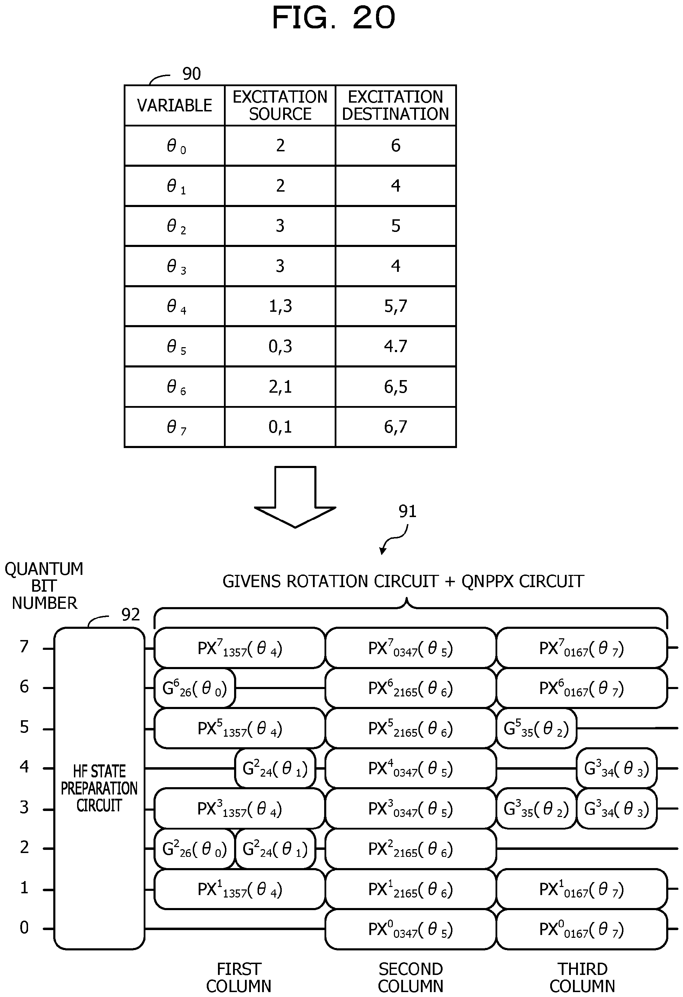

FIG. 20 is a diagram illustrating an example of an Ansatz circuit in which circuits of one-electron excitations and circuits of two-electron excitations coexist in the same column. The quantum circuit generation unit 110 generates an Ansatz circuit 91 while referring to a generation table 90. In the Ansatz circuit 91, a HF state preparation circuit 92 for preparing a HF state is first disposed. Following the HF state preparation circuit 92, a plurality of Givens rotation circuits corresponding to one-electron excitations and QNPPX circuits corresponding to two-electron excitations are disposed in a mixed manner.

For example, the quantum circuit generation unit 110 first disposes, in the first column, a QNPPX circuit that has each quantum bit with quantum bit numbers "1, 3" as an excitation source, and each quantum bit with quantum bit numbers "5, 7" as an excitation destination.

In the QNPPX circuit to be placed next (the sixth row in the generation table 90), "7" is included among the quantum bit numbers of the excitation destinations. Since the quantum bit number "7" is already used in the first column by a QNPPX circuit, the quantum bit number "7" cannot be placed any more in the first column. Therefore, the quantum circuit generation unit 110 disposes, in the second column, a QNPPX circuit that has each quantum bit with quantum bit numbers "0, 3" as an excitation source, and each quantum bit with quantum bit numbers "4, 7" as an excitation destination.

The quantum circuit generation unit 110 next disposes, in the second column, a QNPPX circuit that has each quantum bit with quantum bit numbers "2, 1" as an excitation source, and each quantum bit with quantum bit numbers "6, 5" as an excitation destination.

In the QNPPX circuit to be placed next (the eighth row in the generation table 90), "1" and "7" are included among the quantum bit numbers of the excitation destinations. The quantum bit numbers "1" and "7" are already used by QNPPX circuits both in the first column and the second column. Therefore, the QNPPX circuit to be placed next cannot be disposed in either of the first column and the second column. Therefore, the quantum circuit generation unit 110 disposes, in the third column, a QNPPX circuit that has each quantum bit with quantum bit numbers "0, 1" as an excitation source, and each quantum bit with quantum bit numbers "6, 7" as an excitation destination.

After that, the quantum circuit generation unit 110 disposes Givens rotation circuits in vacant regions in the respective columns. Note that, in the example in FIG. 20, it is assumed that two Givens rotation circuits can be processed within the processing time of a QNPPX circuit. As a result, a Givens rotation circuit that has the quantum bit with the quantum bit number "2" as the excitation source and the quantum bit with the quantum bit number "6" as the excitation destination are placed in the first column. Next, a Givens rotation circuit that has the quantum bit with the quantum bit number "2" as the excitation source and the quantum bit with the quantum bit number "4" as the excitation destination is placed in the same first column. Next, a Givens rotation circuit that has the quantum bit with the quantum bit number "3" as the excitation source and the quantum bit with the quantum bit number "5" as the excitation destination is placed in the third column. Lastly, a Givens rotation circuit that has the quantum bit with the quantum bit number "3" as the excitation source and the quantum bit with the quantum bit number "4" as the excitation destination is placed in the third column.

As one-electron excitation circuits and two-electron excitation circuits can coexist in the same column in this manner, the length of the Ansatz circuit 91 can be shortened.

[Other Embodiments]

In the second embodiment, an example case where the base energy of a nitrogen molecule is calculated has been described. However, the processes described in the second embodiment can also be applied to other types of quantum chemical calculation.

The above description merely indicates the principles of the present invention. Further, numerous modifications and changes may be made by those skilled in the art, and the present invention is not limited to the above-described or illustrated exact configurations and application examples. All corresponding modifications and equivalents are regarded as those within the scope of the present invention by appended claims and equivalents thereof.

REFERENCE SIGNS LIST

1 Quantum circuit
2a, 2b, ···, 3a, 3b, ··· Partial circuit
10 Information processing device
11 Storage unit
12 Processing unit

**Claims**

1. An information processing program for causing a computer to perform a process that includes:

   calculating an initial value of a variable that corresponds to each electron excitation of a plurality of electron excitations to calculate a base energy of a molecule by variational quantum eigensolver;
   determining, for each electron excitation of the plurality of electron excitations, whether to implement a corresponding circuit based on whether the initial value of the corresponding variable is greater than a predetermined threshold; and
   placing a partial circuit that performs a gate operation of the electron excitation to calculate energy in accordance with a value of the variable in a quantum circuit that calculates the base energy of the molecule, for each electron excitation for which it is determined to implement the corresponding circuit among the plurality of electron excitations.

2. The information processing program according to claim 1, further causing the computer to perform a process that includes,
   when the electron excitation for which it is determined to implement the corresponding circuit is a one-electron excitation, placing, in the quantum circuit, the partial circuit that performs a Givens rotation operation that corresponds to the electron excitation.

3. The information processing program according to claim 1 or 2, further causing the computer to perform a process that includes,
   when the electron excitation for which it is determined to implement the corresponding circuit is a two-electron excitation, placing, in the quantum circuit, the partial circuit that performs a quantum-number-preserving-pair-exchange(QNPPX) operation that corresponds to the electron excitation.

4. The information processing program according to any one of claims 1 to 3, further causing the computer to perform a process that includes
   calculating a configuration interaction (CI) coefficient of each electron excitation of the plurality of electron excitations by a classical quantum chemical calculation technique, and setting the calculated CI coefficient as the initial value of the variable of each electron excitation of the plurality of electron excitations, in the process of calculating the initial value of the variable.

5. The information processing program according to any one of claims 1 to 4, wherein
   the process of placing the partial circuit in the quantum circuit includes placing a second partial circuit in a same column as a first partial circuit in the quantum circuit, when quantum bits of an excitation source and an excitation destination in the second partial circuit to be next placed do not overlap any of quantum bits of an excitation source and an excitation destination in the first partial circuit already placed in the column in which circuits that are simultaneously executable in the quantum circuit are placed.

6. The information processing program according to any one of claims 1 to 4, wherein
   the process of placing the partial circuit in the quantum circuit includes: placing a third partial circuit that corresponds to a two-electron excitation in a column in which circuits that are simultaneously executable in the quantum circuit are placed; and placing, in the same column as the third partial circuit, a plurality of fourth partial circuits that corresponds to one-electron excitations that have quantum bits of excitation sources and excitation destinations that do not overlap any of quantum bits of an excitation source and an excitation destination in the third partial circuit, within a range in which the number of times a same quantum bit is used as an excitation source or an excitation destination in the plurality of fourth partial circuits does not exceed a predetermined number.

7. An information processing method for causing a computer to perform a process that includes:

calculating an initial value of a variable that corresponds to each electron excitation of a plurality of electron excitations to calculate a base energy of a molecule by variational quantum eigensolver;

determining, for each electron excitation of the plurality of electron excitations, whether to implement a corresponding circuit based on whether the initial value of the corresponding variable is greater than a predetermined threshold; and

placing a partial circuit that performs a gate operation of the electron excitation to calculate energy in accordance with a value of the variable in a quantum circuit that calculates the base energy of the molecule, for each electron excitation for which it is determined to implement the corresponding circuit among the plurality of electron excitations.

8. An information processing device comprising:
a processing unit configured to:

calculate an initial value of a variable that corresponds to each electron excitation of a plurality of electron excitations to calculate a base energy of a molecule by variational quantum eigensolver;

determine, for each electron excitation of the plurality of electron excitations, whether to implement a corresponding circuit based on whether the initial value of the corresponding variable is greater than a predetermined threshold; and

place a partial circuit that performs a gate operation of the electron excitation to calculate energy in accordance with a value of the variable in a quantum circuit that calculates the base energy of the molecule, for each electron excitation for which it is determined to implement the corresponding circuit among the plurality of electron excitations.

# FIG. 1

INFORMATION PROCESSING DEVICE — 10

PROCESSING UNIT — 12

CALCULATION OF INITIAL VALUE OF VARIABLE OF EACH ELECTRON EXCITATION

$|\psi(\theta)\rangle = a|\psi 0\rangle + b|\psi 1\rangle + c|\psi 2\rangle + \cdots$

| EXCITATION SOURCE | EXCITATION DESTINATION | VARIABLE INITIAL VALUE (CI COEFFICIENT) | |
|---|---|---|---|
| 0 | 4 | $C_{04}$ | ONE-ELECTRON EXCITATIONS |
| 1 | 5 | $C_{15}$ | |
| ... | ... | ... | |
| 0,3 | 4,7 | $C_{0347}$ | TWO-ELECTRON EXCITATIONS |
| 2,1 | 6,5 | $C_{2165}$ | |
| ... | ... | ... | |

DETERMINATION AS TO WHETHER TO IMPLEMENT

· INITIAL VALUE OF VARIABLE ≥ THRESHOLD → TO IMPLEMENT
· INITIAL VALUE OF VARIABLE < THRESHOLD → NOT TO IMPLEMENT

QUANTUM CIRCUIT (USING ROTATION GATE ELEMENTS) — 1

2a 2b

3a 3b

PARTIAL CIRCUIT (ONE-ELECTRON EXCITATION)

PARTIAL CIRCUIT (TWO-ELECTRON EXCITATION)

STORAGE UNIT — 11

# FIG. 2

# FIG. 3

# FIG. 4

**CLASSICAL COMPUTER** — 100

- QUANTUM CIRCUIT GENERATION UNIT — 110
- OPTIMIZATION CALCULATION UNIT — 130
- QUANTUM CALCULATION MANAGEMENT UNIT — 120

**QUANTUM COMPUTER** — 200

# FIG. 5

30

31

|ψ0> —— Ansatz CIRCUIT —— $|\psi(\theta)\rangle = a|\psi 0\rangle + b|\psi 1\rangle + c|\psi 2\rangle + \cdots$

SUPERIMPOSED STATE
EXPRESSED WITH VARIABLE $\theta$

# FIG. 6

40

**Ansatz CIRCUIT (UCCSD)**

41a  41b

ONE-ELECTRON EXCITATION
PARTIAL CIRCUIT

4(j-i) CNOT GATES
10 ONE-QUBIT GATES

42a  42b

TWO-ELECTRON EXCITATION
PARTIAL CIRCUIT

16(j+l-i-k) CNOT GATES
72 ONE-QUBIT GATES

$|\psi 0>$

50

**Ansatz CIRCUIT (USING ROTATION GATE ELEMENT)**

51a  51b

ONE-ELECTRON EXCITATION
PARTIAL CIRCUIT
(GIVENS ROTATION CIRCUIT)

2 TWO-QUBIT GATES
4 ONE-QUBIT GATES

52a  52b

TWO-ELECTRON EXCITATION
PARTIAL CIRCUIT
(QNPPX CIRCUIT)

12 TWO-QUBIT GATES
10 ONE-QUBIT GATES

$|\psi 0>$

# FIG. 7

QUANTUM BIT: a — $G^a_{ab}(\theta)$ — ⟨61⟩

QUANTUM BIT: b — $G^b_{ab}(\theta)$ — ⟨62⟩

= H(63) • $R_Y(\theta)$(65) • H(68)

X(64) $R_Y(\theta)$(66) X(67)

⟨60⟩

MATHEMATICAL EXPRESSION

$$G_{ab}(\theta)|\cdots \underset{b}{0}\cdots \underset{a}{1}\cdots\rangle = \cos\theta|\cdots 0\cdots 1\cdots\rangle + \sin\theta|\cdots 1\cdots 0\cdots\rangle$$

$$G_{ab}(\theta)|\cdots 1\cdots 0\cdots\rangle = \cos\theta|\cdots 1\cdots 0\cdots\rangle - \sin\theta|\cdots 0\cdots 1\cdots\rangle$$

$$G_{ab}(\theta)|\cdots 0\cdots 0\cdots\rangle = |\cdots 0\cdots 0\cdots\rangle$$

$$G_{ab}(\theta)|\cdots 1\cdots 1\cdots\rangle = |\cdots 1\cdots 1\cdots\rangle$$

$$G_{ab}(\theta) = a_b a_a a^\dagger_b a^\dagger_a + a^\dagger_b a^\dagger_a a_b a_a + \cos\theta(a^\dagger_a a_a + a^\dagger_b a_b) + \sin\theta(a^\dagger_b a_a - a^\dagger_a a_b)$$
$$= I + (\cos\theta - 1)(a^\dagger_a a_a + a^\dagger_b a_b) + (\sin\theta)(a^\dagger_b a_a - a^\dagger_a a_b)$$

NUMBER OF GATES

2 TWO-QUBIT GATES FOR ONE EXCITATION
4 ONE-QUBIT GATES FOR ONE EXCITATION

# FIG. 8

# FIG. 9

70

QUANTUM BIT:a1 — $PX^{a1}_{a1a2b1b2}(\theta)$ —

QUANTUM BIT:a2 — $PX^{a2}_{a1a2b1b2}(\theta)$ —

QUANTUM BIT:b1 — $PX^{b1}_{a1a2b1b2}(\theta)$ —

QUANTUM BIT:b2 — $PX^{b2}_{a1a2b1b2}(\theta)$ —

MATHEMATICAL
EXPRESSION

$$PX_{a1a2b1b2}(\theta)|\cdots\overset{b_2}{0}\cdots\overset{b_1}{0}\cdots\overset{a_2}{1}\cdots\overset{a_1}{1}\cdots\rangle=\cos\theta\ |\cdots0\cdots0\cdots1\cdots1\cdots\rangle+\sin\theta\ |\cdots1\cdots1\cdots0\cdots0\cdots\rangle$$

$$\vdots$$

$$PX_{a1a2b1b2}(\theta)=I + (\cos\theta-1)(a^{\dagger}_{a2}a^{\dagger}_{a1}a_{a2}a_{a1} +a^{\dagger}_{b2}a^{\dagger}_{b1}a_{b2}a_{b1})$$
$$+(\sin\theta)(a^{\dagger}_{b2}a^{\dagger}_{b1}a_{a2}a_{a1} -a^{\dagger}_{a2}a^{\dagger}_{a1}a_{b2}a_{b1})$$

NUMBER OF GATES

14 TWO-QUBIT GATES FOR ONE EXCITATION
10 ONE-QUBIT GATES FOR ONE EXCITATION

# FIG. 10

```
        ┌─────────────┐
        │    START    │
        └─────────────┘
               │
               ▼
   ┌───────────────────────┐   S101
   │   DETERMINE PROBLEM   │
   └───────────────────────┘
               │
               ▼
   ┌───────────────────────┐   S102
   │   QUANTUM CIRCUIT     │
   │     GENERATION        │
   └───────────────────────┘
               │
               ▼
   ┌───────────────────────┐   S103
   │ BASE ENERGY CALCULATION│
   └───────────────────────┘
               │
               ▼
   ┌───────────────────────┐   S104
   │ OUTPUT OPTIMAL SOLUTION│
   └───────────────────────┘
               │
               ▼
        ┌─────────────┐
        │     END     │
        └─────────────┘
```

# FIG. 11

START

↓

CALCULATE CI COEFFICIENT — S111

↓

GENERATE INITIAL STATE — S112

↓

EXECUTE LOOP FOR ALL ONE-ELECTRON EXCITATIONS — S113

↓

IS ABSOLUTE VALUE OF CI COEFFICIENT GREATER THAN THRESHOLD? — S114

NO → SET GENERATION FLAG "No" — S116

YES ↓

SET GENERATION FLAG "Yes" — S115

↓

EXECUTE LOOP FOR ALL ONE-ELECTRON EXCITATIONS — S117

↓

A

# FIG. 12

A

EXECUTE LOOP FOR ALL TWO-ELECTRON EXCITATIONS — S121

IS ABSOLUTE VALUE OF CI COEFFICIENT GREATER THAN THRESHOLD? — S122

NO

YES

SET GENERATION FLAG "Yes" — S123

SET GENERATION FLAG "No" — S124

EXECUTE LOOP FOR ALL TWO-ELECTRON EXCITATIONS — S125

GENERATE GIVENS ROTATION CIRCUITS OF ONE-ELECTRON EXCITATIONS ASSOCIATED WITH GENERATION FLAG "Yes", AND INITIALIZE $\theta$ — S126

GENERATE QNPPX CIRCUITS OF TWO-ELECTRON EXCITATIONS ASSOCIATED WITH GENERATION FLAG "Yes", AND INITIALIZE $\theta$ — S127

END

# FIG. 13

80

| VARIABLE | EXCITATION SOURCE | EXCITATION DESTINATION |
|---|---|---|
| $\theta_0$ | 0 | 4 |
| $\theta_1$ | 1 | 5 |
| $\theta_2$ | 0 | 6 |
| $\theta_3$ | 3 | 7 |
| $\theta_4$ | 0,1 | 4,5 |
| $\theta_5$ | 0,3 | 4,7 |
| $\theta_6$ | 2,1 | 6,5 |
| $\theta_7$ | 0,1 | 6,7 |

81

**QUANTUM BIT NUMBER** 82

**GIVENS ROTATION CIRCUIT**  **QNPPX CIRCUIT**

| Bit | | First Column | Second Column | Third Column | Fourth Column | Fifth Column |
|---|---|---|---|---|---|---|
| 7 | HF STATE PREPARATION CIRCUIT | $G^7_{37}(\theta_3)$ | | | $PX^7_{0347}(\theta_5)$ | $PX^7_{0167}(\theta_7)$ |
| 6 | | | $G^6_{06}(\theta_2)$ | | $PX^6_{2165}(\theta_6)$ | $PX^6_{0167}(\theta_7)$ |
| 5 | | $G^5_{15}(\theta_1)$ | | $PX^5_{0145}(\theta_4)$ | $PX^5_{2165}(\theta_6)$ | |
| 4 | | $G^4_{04}(\theta_0)$ | | $PX^4_{0145}(\theta_4)$ | $PX^4_{0347}(\theta_5)$ | |
| 3 | | $G^3_{37}(\theta_3)$ | | | $PX^3_{0347}(\theta_5)$ | |
| 2 | | | | | $PX^2_{2165}(\theta_6)$ | |
| 1 | | $G^1_{15}(\theta_1)$ | | $PX^1_{0145}(\theta_4)$ | $PX^1_{2165}(\theta_6)$ | $PX^1_{0167}(\theta_7)$ |
| 0 | | $G^0_{04}(\theta_0)$ | $G^0_{06}(\theta_2)$ | $PX^0_{0145}(\theta_4)$ | $PX^0_{0347}(\theta_5)$ | $PX^0_{0167}(\theta_7)$ |

FIRST COLUMN  SECOND COLUMN  THIRD COLUMN  FOURTH COLUMN  FIFTH COLUMN

# FIG. 14

$\theta$ UPDATE

# FIG. 15

CLASSICAL
COMPUTER

QUANTUM
COMPUTER

START

S201

INSTRUCT QUANTUM
COMPUTER TO
CALCULATE ENERGY

S202

CALCULATE QUANTUM
CIRCUIT

S203

CALCULATE ENERGY

S204

IS ENERGY
DIFFERENCE BEFORE AND
AFTER UPDATE EQUAL TO OR
SMALLER THAN
THRESHOLD?

NO

YES

S205

OPTIMIZE $\theta$

S206

ACQUIRE BASE ENERGY

END

# FIG. 16

83

LITHIUM HYDROXIDE

ENERGY

-7.60
-7.65
-7.70
-7.75
-7.80
-7.85
-7.90

0.5    1.0    1.5    2.0    2.5    3.0

INTERATOMIC DISTANCE

+ : UCCSD

O : USING ROTATION GATE ELEMENTS

# FIG. 17

84

| Ansatz CIRCUIT | NUMBER OF ONE-QUBIT GATES | RATIO TO UCCSD (ONE-QUBIT GATES) | NUMBER OF TWO-QUBIT GATES | RATIO TO UCCSD (TWO-QUBIT GATES) |
|---|---|---|---|---|
| USING ROTATION GATE ELEMENTS (THRESHOLD: $10^{-2}$) | 610 | 1.5% | 854 | 1.3% |
| USING ROTATION GATE ELEMENTS (THRESHOLD: $10^{-20}$) | 5798 | 14.1% | 7966 | 12.1% |
| UCCSD | 41244 | - | 65968 | - |

# FIG. 18

START

CREATE GENERATION TABLE — S301

Dep=1 — S302

EXECUTE LOOP FOR EACH TWO-ELECTRON EXCITATION x — S303

EXECUTE LOOP FOR EACH COLUMN z FROM FIRST COLUMN TO Dep COLUMN — S304

HAVE QUANTUM BITS OF TWO-ELECTRON EXCITATION x BEEN USED IN COLUMN z? — S305

NO

YES

EXECUTE LOOP FOR EACH COLUMN FROM FIRST COLUMN TO Dep COLUMN — S306

DISPOSE QNPPX CIRCUIT OF TWO-ELECTRON EXCITATION x IN COLUMN z — S307

Dep+1 — S308

DISPOSE QNPPX CIRCUIT OF TWO-ELECTRON EXCITATION x IN "Dep" COLUMN — S309

EXECUTE LOOP FOR EACH TWO-ELECTRON EXCITATION x — S310

B

# FIG. 19

B

S321
EXECUTE LOOP FOR EACH
ONE-ELECTRON EXCITATION y

S322
EXECUTE LOOP FOR EACH
COLUMN z FROM FIRST COLUMN
TO Dep COLUMN

S323
HAVE QUANTUM BITS
OF ONE-ELECTRON EXCITATION y BEEN
USED FOR TWO-ELECTRON EXCITATION
IN COLUMN z?

YES

NO

S324
HAVE QUANTUM BITS
OF ONE-ELECTRON EXCITATION y BEEN
USED FOR ONE-ELECTRON EXCITATION n
TIMES IN COLUMN z?

NO

YES

S325
EXECUTE LOOP FOR EACH COLUMN
FROM FIRST COLUMN TO Dep
COLUMN

S326
DISPOSE GIVENS ROTATION
CIRCUIT OF ONE-ELECTRON
EXCITATION y IN COLUMN z

S327
Dep+1

S328
DISPOSE GIVENS ROTATION
CIRCUIT OF ONE-ELECTRON
EXCITATION y IN "Dep" COLUMN

S329
EXECUTE LOOP FOR EACH ONE-
ELECTRON EXCITATION y

END

# FIG. 20

90

| VARIABLE | EXCITATION SOURCE | EXCITATION DESTINATION |
|---|---|---|
| $\theta_0$ | 2 | 6 |
| $\theta_1$ | 2 | 4 |
| $\theta_2$ | 3 | 5 |
| $\theta_3$ | 3 | 4 |
| $\theta_4$ | 1,3 | 5,7 |
| $\theta_5$ | 0,3 | 4.7 |
| $\theta_6$ | 2,1 | 6,5 |
| $\theta_7$ | 0,1 | 6,7 |

91

QUANTUM BIT NUMBER

GIVENS ROTATION CIRCUIT + QNPPX CIRCUIT

92

| Quantum bit | HF STATE PREPARATION CIRCUIT | FIRST COLUMN | SECOND COLUMN | THIRD COLUMN |
|---|---|---|---|---|
| 7 | | $PX^7_{1357}(\theta_4)$ | $PX^7_{0347}(\theta_5)$ | $PX^7_{0167}(\theta_7)$ |
| 6 | | $G^6_{26}(\theta_0)$ | $PX^6_{2165}(\theta_6)$ | $PX^6_{0167}(\theta_7)$ |
| 5 | | $PX^5_{1357}(\theta_4)$ | $PX^5_{2165}(\theta_6)$ | $G^5_{35}(\theta_2)$ |
| 4 | | $G^2_{24}(\theta_1)$ | $PX^4_{0347}(\theta_5)$ | $G^3_{34}(\theta_3)$ |
| 3 | | $PX^3_{1357}(\theta_4)$ | $PX^3_{0347}(\theta_5)$ | $G^3_{35}(\theta_2)$ $G^3_{34}(\theta_3)$ |
| 2 | | $G^2_{26}(\theta_0)$ $G^2_{24}(\theta_1)$ | $PX^2_{2165}(\theta_6)$ | |
| 1 | | $PX^1_{1357}(\theta_4)$ | $PX^1_{2165}(\theta_6)$ | $PX^1_{0167}(\theta_7)$ |
| 0 | | | $PX^0_{0347}(\theta_5)$ | $PX^0_{0167}(\theta_7)$ |

## INTERNATIONAL SEARCH REPORT

| International application No. |
| --- |
| **PCT/JP2022/011510** |

### A.    CLASSIFICATION OF SUBJECT MATTER

*G06N 10/20*(2022.01)i
FI:    G06N10/20

According to International Patent Classification (IPC) or to both national classification and IPC

### B.    FIELDS SEARCHED

Minimum documentation searched (classification system followed by classification symbols)

G06N10/00-10/80

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C.    DOCUMENTS CONSIDERED TO BE RELEVANT

| Category* | Citation of document, with indication, where appropriate, of the relevant passages | Relevant to claim No. |
| --- | --- | --- |
| A | JP 2020-187451 A (QUNASYS INC) 19 November 2020 (2020-11-19)<br>entire text, all drawings | 1-8 |
| A | 森田 幹雄 ほか, 低回路深さ同時測定型ＶＱＥの計算コスト検証, 情報処理学会 研究報告 量子ソフトウェア（ＱＳ）, 2021, vol. 2021-QS-2, no. 11, pp. 1-7, ISSN 2435-6492<br>entire text, all drawings, (MORITA, Mikio et al. Computational Cost Estimation of Low-depth Simultaneous Measurement Type VQE. IPSJ SIG Technical Report: Quantum Software (QS).) | 1-8 |
| A | XIA, Rongxin et al. Qubit coupled cluster singles and doubles variational quantum eigensolver ansatz for electronic structure calculations. arXiv.org [online]. 2020, pp. 1-13, [retrieval date 24 May 2022], Internet: <URL:https://arxiv.org/abs/2005.08451v3><br>entire text, all drawings | 1-8 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **24 May 2022** | **31 May 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2022/011510**

| Patent document cited in search report | | | Publication date (day/month/year) | Patent family member(s) | | | Publication date (day/month/year) |
|---|---|---|---|---|---|---|---|
| JP | 2020-187451 | A | 19 November 2020 | US | 2021/0183476 | A1 | |
| | | | | entire text, all drawings | | | |
| | | | | WO | 2020/230794 | A1 | |
| | | | | entire text, all drawings | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020230794 A
- US 20190354316
- JP 2020534607 A

**Non-patent literature cited in the description**

- **RONGXIN XIA** ; **SABRE KAIS**. Qubit coupled cluster singles and doubles variational quantum eigensolver ansatz for electronic structure calculations. *arXiv:2005.08451v3*, 10 October 2020